(19) Europäisches Patentamt
European Patent Office
Office européen des brevets

(11) **EP 3 160 580 B1**

(12) **EUROPÄISCHE PATENTSCHRIFT**

(45) Veröffentlichungstag und Bekanntmachung des Hinweises auf die Patenterteilung:
**15.11.2023 Patentblatt 2023/46**

(21) Anmeldenummer: **15732639.8**

(22) Anmeldetag: **26.06.2015**

(51) Internationale Patentklassifikation (IPC):
**A61N 1/375** (2006.01)  **A61N 1/04** (2006.01)
**A61N 1/36** (2006.01)

(52) Gemeinsame Patentklassifikation (CPC):
**A61N 1/362; A61N 1/3754;** A61N 1/04

(86) Internationale Anmeldenummer:
**PCT/EP2015/064493**

(87) Internationale Veröffentlichungsnummer:
**WO 2015/197810 (30.12.2015 Gazette 2015/52)**

(54) **KABELLOSER HERZSCHRITTMACHER MIT CERMET-ELEKTRODE UND HERSTELLUNGSVERFAHREN DAFÜR**

WIRELESS CARDIAC PACEMAKER WITH CERMET ELECTRODE AND MANUFACTURING METHOD THEREFOR

STIMULATEUR CARDIAQUE SANS FIL COMPORTANT UNE ÉLECTRODE CERMET Y PROCEDE DE FABRICATION ASSOCIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.06.2014 DE 102014009322**

(43) Veröffentlichungstag der Anmeldung:
**03.05.2017 Patentblatt 2017/18**

(73) Patentinhaber: **Heraeus Deutschland GmbH & Co. KG**
**63450 Hanau (DE)**

(72) Erfinder:
• **SCHIBLI, Stefan**
 **60326 Frankfurt (DE)**
• **TRÖTZSCHEL, Jens**
 **63549 Ronneburg (DE)**

(74) Vertreter: **Herzog IP Patentanwalts GmbH**
**Steinstraße 16-18**
**40212 Düsseldorf (DE)**

(56) Entgegenhaltungen:
**US-A1- 2011 034 965  US-A1- 2012 193 119**
**US-A1- 2012 194 981  US-A1- 2013 338 750**
**US-B1- 8 000 804**

EP 3 160 580 B1

**Beschreibung**

Die Erfindung, welche in den Ansprüchen definiert ist, bezieht sich auf eine Vorrichtung, beinhaltend einen Hohlkörper, ein Innenvolumen und ein Umgebungsvolumen, wobei der Hohlkörper eine Cermet-Elektrode beinhaltet; auf ein Verfahren zu einer Herstellung einer Vorrichtung, beinhaltend als Verfahrensschritt ein Verbinden eines Gehäuses mit einer Cermet-Elektrode; eine Vorrichtung, erhältlich durch ein Verfahren, beinhaltend als Verfahrensschritt ein Verbinden eines Gehäuses mit einer Cermet-Elektrode; und auf eine Verwendung einer Cermet-Elektrode zu einem Herstellen eines kabellosen Herzschrittmachers.

Die elektrische Stimulation menschlichen oder tierischen Gewebes zu therapeutischen Zwecken ist im Stand der Technik seit Langem bekannt. Für eine solche Therapie kann ein elektrische Impulse gebendes Gerät in einen zu therapierenden menschlichen oder tierischen Organismus implantiert werden. Beispielhafte implantierbare elektrische Impulse gebende Geräte sind Schrittmacher und Defibrillatoren. Im Stand der Technik bekannte Schrittmacher sind Blasenschrittmacher, Zwerchfellschrittmacher, Darmschrittmacher, Atemschrittmacher, Hirnschrittmacher und Herzschrittmacher. Im Stand der Technik seit Längerem bekannte implantierbare Herzschrittmacher beinhalten typischerweise eine Steuereinheit, welche verbunden ist mit einer Messeinheit und einem elektrischen Impulsgenerator. Die Messeinheit erfasst über angeschlossene Elektroden elektrische Potentiale des Herzens. Der elektrische Impulsgenerator gibt elektrische Stimulationsimpulse über angeschlossene Elektroden an ein Herzmuskelgewebe ab. Herkömmliche seit Längerem im Stand der Technik bekannte Herzschrittmacher werden in einiger Entfernung vom Herzen implantiert, beispielsweise beim Menschen unterhalb des Schlüsselbeins. Von der im Gehäuse beinhalteten Messeinheit und dem elektrischen Impulsgenerator führen flexible Elektrodenleitungen in eine Herzkammer. Dort enden die Elektrodenleitungen an Elektroden, welche die elektrischen Stimulationsimpulse an das Herzmuskelgewebe übertragen. Die Energieversorgung der Steuereinheit wird dabei über eine ebenfalls implantierte Batterie realisiert. Die ebenfalls implantierte Batterie kann in dem Gehäuse beinhaltet sein oder mit diesem durch eine Stecker-Buchse-Verbindung verbunden sein. Zahlreiche Weiterentwicklungen solcher Herzschrittmacher betreffen verwendete Materialien, Programmierungen der Steuereinheit sowie die Energieversorgung. Aktuelle im Stand der Technik bekannte Herzschrittmacher beinhalten die flexiblen Elektrodenleitungen zwischen der Messeinheit und Elektroden sowie zwischen dem elektrischen Impulsgenerator und Elektroden nicht. Vielmehr ist das Gehäuse so ausgebildet, dass es die Elektroden an seinen Außenseiten beinhaltet. Durch eine entsprechende Dimensionierung eines solchen Herzschrittmachers kann dieser in ein Herz implantiert werden, wo die Elektroden in Kontakt mit einem Herzmuskelgewebe stehen. Herzschrittmacher dieser Art werden vom Fachmann kabellose Herzschrittmacher genannt. Ein solcher kabelloser Herzschrittmacher ist in EP 1 714 670 A1 offenbart. Ein kabelloser Herzschrittmacher beinhaltet mindestens zwei Elektroden. Eine Elektrode ist als Kathode ausgebildet und eine weitere Elektrode ist als Anode ausgebildet. Die Kathode ist oft als Tip-Elektrode ausgebildet und die Anode als Ring-Elektrode. Die Kathode dient zur Stimulation des Herzmuskels und die Anode zur Messung der Herzaktivität. Als Anode wird oft herkömmlich das Gehäuse, welches aus Titan besteht, verwendet. Die Kathode besteht herkömmlich aus einem Kontaktkörper und einem Durchführungsdraht. Der Kontaktkörper ist ausgebildet zum Kontaktieren des zu stimulierenden Gewebes. Herkömmlich besteht der Kontaktkörper aus einer Platin-Iridium-Legierung und wird durch Zerspanen hergestellt. Der Durchführungsdraht ist an den Kontaktkörper angeschweißt und stellt eine elektrische Verbindung zwischen der Elektronik innerhalb des Gehäuses und dem Kontaktkörper her. Von großer Wichtigkeit für die Implantierbarkeit des Herzschrittmachers ist hierbei, dass die elektrische Durchführung hermetisch dicht ist. Um dies zu erreichen, führt herkömmlich der Durchführungsdraht durch einen Keramikring, mit dem der Durchführungsdraht durch ein Gold-Lot verlötet ist. Der Keramikring wiederum ist in einen Metallflansch eingelötet, welcher durch Schweißen an das Gehäuse angeflanscht ist.

Die kabellosen Herzschrittmacher des Stands der Technik beinhalten mindestens folgende Nachteile. Eine Elektrode besteht aus mehreren Teilen, mindestens dem Kontaktkörper und dem Durchführungsdraht. Diese Teile müssen in mindestens einem Arbeitsschritt verbunden werden. Dieser Arbeitsschritt gestaltet ein Herstellungsverfahren eines kabellosen Herzschrittmachers des Stands der Technik teurer oder aufwendiger oder beides. Eine Elektrode eines kabellosen Herzschrittmachers des Stands der Technik ist mehrstückig ausgebildet. Dies erhöht den elektrischen Widerstand gegenüber einer einstückigen Elektrode, was den Energieverbrauch eines kabellosen Herzschrittmachers des Stands der Technik erhöht. Der erhöhte Energieverbrauch verkürzt die Batterielebensdauer eines kabellosen Herzschrittmachers des Stands der Technik. Dies verkürzt die Dauer zwischen notwendigen chirurgischen Eingriffen in einen Organismus, in den der kabellose Herzschrittmacher des Stands der Technik implantiert ist. Die Verbindung der Teile der Elektrode des kabellosen Herzschrittmachers des Stands der Technik kann sich lösen, was zu einem Beeinträchtigen der Funktion des kabellosen Herzschrittmachers führen kann oder den Organismus, in den der kabellose Herzschrittmacher implantiert ist, schädigen kann. Zudem muss der Durchführungsdraht mit dem Keramikring in einem zusätzlichen Arbeitsschritt verbunden werden. Auch dieser Arbeitsschritt gestaltet ein Herstellungsverfahren eines kabellosen Herzschrittmachers des Stands der Technik teurer oder aufwendiger oder beides. Das Verbinden des Durchführungsdrahts mit dem Keramikring wird im Stand der Technik durch Löten mit einem Gold-Lot realisiert. Der Einsatz des Gold-Lots gestaltet die Herstellung eines kabellosen Herzschrittmachers des Stands der Technik teurer. Auch die Verbindung des Keramikrings mit dem Durchführungsdraht des kabellosen Herzschrittmachers des Stands der Technik kann sich lösen, was zu einem Beeinträchtigen der Funktion des kabellosen Herzschrittmachers führen kann oder den Organismus, in den der kabellose Herzschrittmacher implantiert ist, schädigen kann. Ferner stellt die Verbindung des Keramikrings mit dem Durchführungsdraht eine potentielle Quelle einer Undichtigkeit des kabellosen Herzschrittmachers des Stands der Technik dar. Ferner ist die Herstellung eines Kontaktkörper eines kabellosen Herzschrittmachers des Stands der Technik aufwendig oder teuer oder beides. Es ist von Vorteil die äußere (makroskopische) Oberfläche des Kontaktkörper, welche zur Stimulation in elektrisch leitenden Kontakt mit dem Herzmuskelgewebe gebracht wird, zu minimieren. Andererseits darf die äußere Oberfläche des Kontaktkörpers, welche in mechanischem Kontakt zu dem Herzmuskelgewebe steht, nicht so klein gewählt werden, dass die Elektrode das Herzmuskelgewebe perforiert. Demnach wird im Stand der Technik die Oberfläche des Kontaktkörper, welche in Kontakt mit dem Herzmuskelgewebe gebracht wird, teilweise elektrisch isolierend beschichtet, zum Beispiel mit einem Parylen. Ein solches Beschichten fügt einem Herstellungsprozess eines kabellosen Herzschrittmachers des Stands der Technik einen zusätzlichen Arbeitsschritt hinzu. Ein solcher Arbeitsschritt gestaltet ein Herstellungsverfahren eines kabellosen Herzschrittmachers des Stands der Technik teurer oder aufwendiger oder beides. Ferner kann sich eine solche Beschichtung oder Teile einer solchen Beschichtung lösen, was zu einem Beeinträchtigen der Funktion des kabellosen Herzschrittmachers führen kann oder den Organismus, in den der kabellose Herzschrittmacher implantiert ist, schädigen kann. Im Weiteren bietet eine möglichst große innere (mikroskopische) Oberfläche des Kontaktkörpers, welche in Kontakt zu dem Herzmuskelgewebe steht Vorteile für den Betrieb eines kabellosen Herzschrittmachers. Im Stand der Technik wird diese Oberfläche deshalb gesintert oder beschichtet oder beides. Dies fügt einem Herstellungsverfahren eines kabellosen Herzschrittmachers aufwendige oder teure oder beides Arbeitsschritte hinzu. Zudem kann sich auch eine solche Beschichtung oder Teile einer solchen Beschichtung lösen, was zu einem Beeinträchtigen der Funktion des kabellosen Herzschrittmachers führen kann oder den Organismus, in den der kabellose Herzschrittmacher implantiert ist, schädigen kann. In einem kabellosen Herzschrittmacher des Stands der Technik wird das Gehäuse als Anode verwendet. Es ist vorteilhaft eine innere (mikroskopische) Oberfläche eines Teils des Gehäuses zu erhöhen. Dies wird im Stand der Technik durch Sintern oder Beschichten oder beides realisiert. Dies fügt einem Herstellungsverfahren eines kabellosen Herzschrittmachers aufwendige oder teure oder beides Arbeitsschritte hinzu. Zudem kann sich auch eine solche Beschichtung oder Teile einer solchen Beschichtung lösen, was zu einem Beeinträchtigen der Funktion des kabellosen Herzschrittmachers führen kann oder den Organismus, in den der kabellose Herzschrittmacher implantiert ist, schädigen kann. Zudem erschwert die Verwendung des Gehäuses als Anode ein Fixieren einer Elektronik in dem Gehäuse ohne einen elektrischen Kurzschluss mit dem Gehäuse zu verursachen. Da die Anode das gesamte Gehäuse ist, wird eine Messung der Herzaktivität leicht durch Störsignale beeinträchtigt. Soll eine elektrische Stimulation oder eine Messung von elektrischen Potentialen an verschiedenen eng beieinanderliegenden Punkten eines Herzmuskelgewebes separat erfolgen, ist eine Anordnung einer Vielzahl von Elektroden dicht beieinander von Vorteil. Eine solche Anordnung ist mit Elektroden von kabellosen Herzschrittmachern des Stands der Technik nicht oder nur unter großem Aufwand möglich. Ein grundsätzliches Problem für ein Implantieren von kabellosen Herzschrittmachern stellen Entzündungen dar. Gegen ein Entstehen von Entzündungen durch den implantierten kabellosen Herzschrittmacher können steroidale Entzündungshemmer eingesetzt werden. Diese werden im Stand der Technik in eine Bohrung in einer Elektrode des kabellosen Herzschrittmachers eingebracht. Eine Vielzahl solcher Bohrungen, welche steroidale Entzündungshemmer beinhalten, wäre vorteilhaft. Ein Erzeugen einer Vielzahl von Bohrungen in eine Elektrode eines kabellosen Herzschrittmachers des Stands der Technik ist sehr aufwendig. US 2013/0338750 A1 offenbart ein implantierbares medizinisches Gerat mit einer Cermetelektrode.

Allgemein ist es eine Aufgabe der vorliegenden Erfindung, einen Nachteil, der sich aus dem Stand der Technik ergibt, zumindest teilweise zu überwinden. Eine Aufgabe der Erfindung ist es einen kabellosen Herzschrittmacher oder einen implantierbaren Biomonitor oder beides bereitzustellen, welcher weniger aufwendig oder günstiger oder beides herzustellen ist. Eine weitere Aufgabe der Erfindung ist es ein weniger aufwendigeres oder günstigeres oder beides Herstellungsverfahren eines kabellosen Herzschrittmachers oder eines implantierbaren Biomonitors oder beides bereitzustellen. Eine weitere Aufgabe der Erfindung ist es einen kabellosen Herzschrittmacher oder einen implantierbaren Biomonitor oder beides mit einem erhöhten Wirkungsgrad bereitzustellen. Eine weitere Aufgabe der Erfindung ist es einen kabellosen Herzschrittmacher oder einen implantierbaren Biomonitor oder beides mit einem geringeren Energieverbrauch bereitzustellen. Eine weitere Aufgabe der Erfindung ist es einen kabellosen Herzschrittmacher oder einen implantierbaren Biomonitor oder beides mit einer längeren Batterielebensdauer bereitzustellen. Eine weitere Aufgabe der Erfindung ist es einen kabellosen Herzschrittmacher oder einen implantierbaren Biomonitor oder beides mit einer erhöhten Betriebssicherheit bereitzustellen. Eine weitere Aufgabe der Erfindung ist es einen kabellosen Herzschrittmacher oder einen implantierbaren Biomonitor oder beides, beinhaltend ein Elektrodenarray bereitzustellen. Eine weitere Aufgabe der Erfindung ist es einen kabellosen Herzschrittmacher, welcher ein ortspräziseres Stimulieren ermöglicht bereitzustellen. Eine weitere Aufgabe der Erfindung ist es einen kabellosen Herzschrittmacher oder einen implantierbaren Biomonitor oder beides, welcher ein ortspräziseres Messen von elektrischen Potentialen ermöglicht bereitzustellen. Eine weitere Aufgabe der Erfindung ist es einen kabellosen Herzschrittmacher mit einer minimalen elektrisch leitenden äußeren Stimulationsoberfläche bereitzustellen. Eine weitere Aufgabe der Erfindung ist es einen kabellosen Herzschrittmacher oder einen implantierbaren Biomonitor oder beides mit einer maximalen Elektrodenoberfläche bereitzustellen. Eine weitere Aufgabe der Erfindung ist es einen kabellosen Herzschrittmacher oder einen implantierbaren Biomonitor oder beides mit einer Vielzahl von Hohlräumen für steroidale Entzündungshemmer bereitzustellen. Eine weitere Aufgabe der Erfindung ist es einen kabellosen Herzschrittmacher oder einen implantierbaren Biomonitor oder beides, welcher nach einem implantieren in einen eukaryotischen Organismus weniger häufig oder weniger schwerwiegende oder beides Entzündungen verursacht bereitzustellen. Eine weitere Aufgabe der Erfindung ist es einen kabellosen Herzschrittmacher oder einen implantierbaren Biomonitor oder beides mit einer verringerten Anzahl von potentiellen Leckstellen bereitzustellen. Eine weitere Aufgabe der Erfindung ist es einen kabellosen Herzschrittmacher oder einen implantierbaren Biomonitor oder beides mit einer verringerten Anzahl von Verbindungsstellen bereitzustellen. Eine weitere Aufgabe der Erfindung ist es einen kabellosen Herzschrittmacher oder einen implantierbaren Biomonitor oder beides mit einer Elektrode, die aus möglichst wenigen Stücken besteht, bereitzustellen. Eine weitere Aufgabe der Erfindung ist es einen kabellosen Herzschrittmacher oder einen implantierbaren Biomonitor oder beides, der möglichst sicher und möglichst dauerhaft hermetisch dicht ist bereitzustellen. Eine weitere Aufgabe der Erfindung ist es einen kabellosen Herzschrittmacher oder einen implantierbaren Biomonitor oder beides mit einer erhöhten Funktionsdauer bereitzustellen. Eine weitere Aufgabe der Erfindung ist es einen weniger störanfälligen kabellosen Herzschrittmacher oder einen implantierbaren Biomonitor oder beides bereitzustellen. Eine weitere Aufgabe der Erfindung ist es ein Herstellungsverfahren für einen der obigen vorteilhaften kabellosen Herzschrittmacher oder einen implantierbaren Biomonitor oder beides bereitzustellen. Eine weitere Aufgabe der Erfindung ist es einen verbesserten kabellosen Herzschrittmacher oder einen implantierbaren Biomonitor oder beides bereitzustellen. Eine weitere Aufgabe der Erfindung ist es eine verbesserte Therapie von Bradykardie bereitzustellen. Eine weitere Aufgabe der Erfindung ist es eine kostengünstigere Therapie von Bradykardie bereitzustellen. Eine weitere Aufgabe der Erfindung ist es eine schonendere Therapie von Bradykardie bereitzustellen. Eine weitere Aufgabe der Erfindung ist es eine Therapie, welche weniger chirurgische Eingriffe beinhaltet, bereitzustellen.

Eine weitere Aufgabe der Erfindung ist es eine risikoärmere Therapie von Bradykardie bereitzustellen.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen.

Einen Beitrag zur Erfüllung mindestens einer der erfindungsgemäßen Aufgaben leistet eine Vorrichtung, beinhaltend einen Hohlkörper, ein Innenvolumen und ein Umgebungsvolumen; wobei

    a) das Innenvolumen ein elektronisches Bauteil beinhaltet;
    b) wobei der Hohlkörper

        i) das Innenvolumen umschließt,
        ii) ein erstes Bauteil, ein zweites Bauteil und eine Elektrode beinhaltet;

    c) das erste Bauteil elektrisch leitfähig ist;
    d) das zweite Bauteil die Elektrode elektrisch von dem ersten Bauteil isoliert;
    e) die Elektrode

        i) ein Cermet beinhaltet,
        ii) das Innenvolumen elektrisch leitend mit dem Umgebungsvolumen verbindet,
        iii) eine Kontaktoberfläche beinhaltet;

    f) die Kontaktoberfläche

        i) ausgebildet ist zu einem Kontaktieren eines eukaryotischen Gewebes,
        ii) einen maximalen Abstand zu dem elektronischen Bauteil von weniger als 80 mm, bevorzugt weniger als 70 mm, bevorzugter weniger als 60 mm, bevorzugter weniger als 50 mm, bevorzugter weniger als 45 mm, bevorzugter weniger als 40 mm, bevorzugter weniger als 35 mm, bevorzugter weniger als 30 mm, bevorzugter weniger als 25 mm, bevorzugter weniger als 20 mm, bevorzugter weniger als 15 mm, noch bevorzugter weniger als 10 mm, am bevorzugtesten weniger als 5 mm, hat.

Bevorzugt verbindet die Elektrode das Innenvolumen elektrisch leitend mit dem Umgebungsvolumen über einen ohmschen Widerstand von nicht mehr als 10 $\Omega$, bevorzugt nicht mehr als 1 $\Omega$, bevorzugter nicht mehr als 200 m$\Omega$, noch bevorzugter nicht mehr als 100 m$\Omega$, am bevorzugtesten nicht mehr 50 m$\Omega$.

In einer erfindungsgemäßen Ausführungsform ist das elektronische Bauteil ein elektrischer Impulsgenerator.

In einer erfindungsgemäßen Ausführungsform ist das elektronische Bauteil eines ausgewählt aus der Gruppe bestehend aus einem Datenspeicher, einer Datenverarbeitungseinheit, einer Energiequelle, einem Aufnahmegerät, und einer Sendeeinrichtung, oder eine Kombination von mindestens zwei davon. Als Datenspeicher kann jede Einheit zum Speichern von Daten ausgewählt werden, welche der Fachmann für geeignet zum Speichern medizinischer Daten, bevorzugt EKG-Daten, in einem implantierbaren Gerät hält. Ein bevorzugter Datenspeicher ist ein Magnetspeicher oder ein Flashspeicher oder beides. Eine bevorzugte Energiequelle ist eine Batterie oder ein Akkumulator oder beides. Ein bevorzugtes Aufnahmegerät ist ein Gerät zu einem Aufnehmen medizinischer Daten, bevorzugt Kardiodaten, bevorzugter EKG-Daten. Ein besonders bevorzugtes Aufnahmegerät ist ein Biomonitor. Ein bevorzugter Biomonitor beinhaltet eines ausgewählt aus der Gruppe bestehend aus einem EKG-Gerät, einem Holter-Monitor, einem Event-Recorder, und einem Loop-Recorder, oder eine Kombination von mindestens zwei davon. Ein bevorzugtes EKG-Gerät ist ein Langzeit-EKG-Gerät, welches bevorzugt Daten speichert, die über einen Zeitraum von mindestens einer Stunde anfallen. Eine bevorzugte Sendeeinrichtung ist ausgebildet zu einem drahtlosen, bevorzugt telemetrischen, Übertragen von Daten, bevorzugt EKG-Daten. Ein bevorzugtes drahtloses Übertragen von Daten ist ein Übertragen mittels Wellen. Bevorzugte Wellen sind Longitudinalwellen oder Transversalwellen oder beides. Bevorzugte Longitudinalwellen sind akustische Wellen oder Schallwellen oder beides. Bevorzugte Transversalwellen sind elektromagnetische Wellen. Bevorzugte elektromagnetische Wellen sind Wellen der Frequenz eines Mobilfunknetzes oder von Bluetooth oder beides. Ein bevorzugtes Mobilfunknetz ist ein GSM-Netz.

In einer erfindungsgemäßen Ausführungsform ist die Elektrode als starrer Körper ausgebildet.

In einer erfindungsgemäßen Ausfuhrungsform ist die Elektrode einstückig ausgebildet. Eine Elektrode ist einstückig ausgebildet, wenn alle zu der Elektrode gehörenden Teile in einem Herstellungsverfahren als ein Stück hergestellt wurden, ohne ein Zusammenfügen vorgefertigter Teile. Eine bevorzugte einstückige Elektrode beinhaltet keine Lötstellen oder keine Schweißstellen oder beides.

In einer erfindungsgemäßen Ausführungsform beinhaltet das Cermet ein Metall zu mindestens 25 Vol.-%, bevorzugt zu mindestens 32 Vol.-%, am bevorzugtesten zu mindestens 38 Vol.-%, basierend auf dem Volumen der Elektrode.

In einer erfindungsgemäßen Ausführungsform beinhaltet das zweite Bauteil eine Keramik.

In einer erfindungsgemäßen Ausführungsform trennt der Hohlkörper das Innenvolumen hermetisch dicht von dem Umgebungsvolumen.

In einer erfindungsgemäßen Ausführungsform beinhaltet der Hohlkörper ein Fixierelement, wobei das Fixierelement ausgebildet ist zu einem Fixieren einer Position des Hohlkörpers relativ zu einem eukaryotischen Gewebe. Fixieren bedeutet hierbei, dass die Bewegung des Hohlkörpers relativ zu dem eukaryotischen Gewebe zumindest vorübergehend, bevorzugt dauerhaft, eingeschränkt wird. Ein bevorzugtes Fixierelement ist eines ausgewählt aus der Gruppe bestehend aus einem Haken, einem Anker und einer Schraube oder eine Kombination von mindestens zwei davon. Ein bevorzugter Hohlkörper beinhaltet mehr als ein Fixierelement.

In einer erfindungsgemäßen Ausführungsform besitzt der Hohlkörper entlang einer kartesischen Raumrichtung eine längste Ausdehnung, wobei die längste Ausdehnung weniger als 150 mm, bevorzugt weniger als 125 mm, bevorzugter weniger als 120 mm, bevorzugter weniger als 115 mm, bevorzugter weniger als 110 mm, bevorzugter weniger als 105 mm, bevorzugter weniger als 100 mm, bevorzugter weniger als 95 mm, bevorzugter weniger als 90 mm, bevorzugter weniger als 85 mm, bevorzugter weniger als 80 mm, bevorzugter weniger als 75 mm, bevorzugter weniger als 70 mm, bevorzugter weniger als 65 mm, bevorzugter weniger als 60 mm, bevorzugter weniger als 55 mm, bevorzugter weniger als 50 mm, bevorzugter weniger als 45 mm, bevorzugter weniger als 40 mm, noch bevorzugter weniger als 35 mm, am bevorzugtesten weniger als 30 mm beträgt.

Ein bevorzugter Hohlkörper ist längserstreckt. Eine bevorzugte längste Ausdehnung eines Hohlkörpers entlang einer kartesischen Raumrichtung ist eine Länge des Hohlkörpers.

In einer erfindungsgemäßen Ausführungsform nimmt der Hohlkörper ein Volumen von höchstens 10 cm$^3$, bevorzugt höchstens 9 cm$^3$, bevorzugter höchstens 8 cm$^3$, bevorzugter höchstens 7 cm$^3$, noch bevorzugter höchstens 6 cm$^3$, am bevorzugtesten höchstens 5 cm$^3$ ein.

Dabei setzt sich das Volumen, welches der Hohlkörper einnimmt zusammen aus dem Innenvolumen und Volumina aller Bestandteile des Hohlkörpers. Bevorzugte Bestandteile des Hohlkörpers sind ausgewählt aus der Gruppe bestehend aus einem Gehäuse, mindestens einer Elektrode, einem Fixierelement, einem Verschluss, einem Flansch, einem Rahmen oder eine Kombination aus mindestens zwei davon.

In einer erfindungsgemäßen Ausführungsform beinhaltet die Elektrode mindestens zwei Teilelektroden, wobei jede der Teilelektroden

a) ein Cermet beinhaltet,

b) das Innenvolumen elektrisch leitend mit dem Umgebungsvolumen verbindet,

c) von der jeweils anderen Teilelektrode oder den jeweils anderen Teilelektroden elektrisch isoliert ist.

Bevorzugt beinhaltet die Elektrode ein Array von Teilelektroden. Ein Array ist hierbei eine zweidimensionale Verteilung einer Vielzahl von Teilelektroden. Ein Array beinhaltet bevorzugt mindestens drei, bevorzugter mindestens vier, bevorzugter mindestens fünf, noch bevorzugter mindestens sechs, am bevorzugtesten mindestens sieben, Teilelektroden.

Eine bevorzugte Teilelektrode ist einstückig ausgebildet. Eine Teilelektrode ist einstückig ausgebildet, wenn alle zu der Teilelektrode gehörenden Teile in einem Herstellungsverfahren als ein Stück hergestellt wurden, ohne ein Zusammenfügen vorgefertigter Teile. Eine bevorzugte einstückige Teilelektrode beinhaltet keine Lötstellen oder keine Schweißstellen oder beides. Eine weitere bevorzugte Teilelektrode ist als starrer Körper ausgebildet. Eine weitere bevorzugte Teilelektrode verbindet das Innenvolumen elektrisch leitend mit dem Umgebungsvolumen über einen ohmschen Widerstand von nicht mehr als 10 Ω, bevorzugt nicht mehr als 1 Ω, bevorzugter nicht mehr als 200 mΩ, noch bevorzugter nicht mehr als 100 mΩ, am bevorzugtesten nicht mehr 50 mΩ.

In einer erfindungsgemäßen Ausführungsform beinhaltet die Kontaktoberfläche eine elektrisch leitende Teiloberfläche, wobei die elektrisch leitende Teiloberfläche eine äußere Oberfläche von kleiner als 25 mm$^2$, bevorzugt kleiner als 15 mm$^2$, bevorzugter kleiner als 10 mm$^2$, am bevorzugtesten kleiner als 5 mm$^2$, hat.

In einer erfindungsgemäßen Ausführungsform beinhaltet die Kontaktoberfläche eine elektrisch leitende Teiloberfläche, wobei eine innere Oberfläche der elektrisch leitenden Teiloberfläche mindestens doppelt so groß ist wie die äußere Oberfläche der elektrisch leitenden Teiloberfläche. Eine bevorzugte innere Oberfläche einer Teiloberfläche der Kontaktoberfläche wurde durch ein Wegätzen einer Keramikmatrix vergrößert. Bevorzugt ist eine innere Oberfläche einer Teiloberfläche der Kontaktoberfläche möglichst groß, damit die elektrische Kapazität der Elektrode möglichst groß ist. Dies führt bevorzugt dazu, dass eine erhöhte Spannung nach einem elektrischen Impuls nach einem kürzeren Zeitraum wieder abgefallen ist. Dies ist bevorzugt vorteilhaft für eine Messung einer Herzaktivität. Eine bevorzugte Kontaktoberfläche beinhaltet eine elektrisch leitende Teiloberfläche, wobei die elektrisch leitende Teiloberfläche eine innere Oberfläche von mindestens 10 mm$^2$, bevorzugt mindestens 20 mm$^2$, bevorzugter mindestens 30 mm$^2$, am bevorzugtesten mindestens 50 mm$^2$, hat.

In einer erfindungsgemäßen Ausführungsform beinhaltet die Kontaktoberfläche eine elektrisch isolierende Teiloberfläche, wobei die elektrisch isolierende Teiloberfläche eine äußere Oberfläche von mehr als 1 mm$^2$, bevorzugt mehr als 1,5 mm$^2$, bevorzugter mehr als 2 mm$^2$, am bevorzugtesten mehr als 5 mm$^2$, hat. Bevorzugt ist eine äußere Oberfläche einer Teiloberfläche der Kontaktoberfläche so groß, dass die Elektrode ein eukaryotisches Gewebe nicht perforiert.

In einer erfindungsgemäßen Ausführungsform hat die Kontaktoberfläche oder eine Teiloberfläche der Kontaktoberfläche oder beide eine mittlere Rauheit Ra in einem Bereich von 0,2 bis 8 μm, bevorzugt in einem Bereich von 0,3 bis 8 μm, bevorzugter in einem Bereich von 0,5 bis 8 μm, am bevorzugtesten in einem Bereich von 1 bis 7 μm.

In einer erfindungsgemäßen Ausführungsform hat die Kontaktoberfläche oder eine Teiloberfläche der Kontaktoberfläche oder beide eine offene Porendichte in einem Bereich von 1000 bis 80000 ppi, bevorzugt in einem Bereich von 2000 bis 60000 ppi, bevorzugter in einem Bereich von 3000 bis 50000 ppi , am bevorzugtesten in einem Bereich von 4000 bis 40000 ppi. Bevorzugt ist eine offene Porendichte der Kontaktoberfläche oder der Teiloberfläche der Kontaktoberfläche oder beider durch ein Ätzen der entsprechenden Oberfläche vergrößert. Bei einem bevorzugten Ätzen wird ein keramischer Anteil des Cermets weggeätzt, was zu einer Porenbildung führt, und ein metallischer Anteil des Cermets bleibt bestehen. Vorzugsweise führt dieses Wegätzen des keramischen Anteils zu einem Metallschaum. Die erfindungsgemäße Kontaktoberfläche oder die Teiloberfläche der Kontaktoberfläche oder beide können mit einem biokompatiblen Metall oder einer biokompatiblen Metallverbindung oder mit beidem beschichtet sein. Ein bevorzugtes biokompatibles Metall ist Iridium. Eine bevorzugte biokompatible Metallverbindung ist eine biokompatible Metalllegierung. Eine weitere bevorzugte Metallverbindung ist Titannitrid (TiN). Bevorzugt wird eine elektrische Kapazität der Elektrode durch das vorgenannte Beschichten der Kontaktoberfläche mit dem Metall oder der Metallverbindung oder beidem erhöht. Bevorzugt wird die elektrische Kapazität auf mindestens 1 mF/cm$^2$, bevorzugter auf mindestens 2 mF/cm$^2$, bevorzugter auf mindestens 3 mF/cm$^2$, bevorzugter auf mindestens 4 mF/cm$^2$, am bevorzugtesten auf mindestens 5 mF/cm$^2$, bezogen auf die Fläche der Kontaktoberfläche erhöht. Bevorzugt wird die elektrische Kapazität wie vorgenannt erhöht, um einen Spannungspulsübertrag von der Elektrode auf ein eukaryotisches Gewebe zu verbessern oder eine Detektion eines elektrischen Signals von einem eukaryotischen Gewebe zu der Elektrode zu verbessern oder beides.

In einer erfindungsgemäßen Ausführungsform beinhaltet die Elektrode einen Hohlraum; wobei der Hohlraum

    a) zu dem Umgebungsvolumen hin offen ist, und
    b) einen Wirkstoff beinhaltet.

Ein bevorzugter Wirkstoff ist ein Entzündungshemmer. Ein bevorzugter Entzündungshemmer ist ausgewählt aus der Gruppe bestehend aus einem steroidalen Entzündungshemmer, einem nichtsteroidalen Entzündungshemmer und einem pflanzlichen Entzündungshemmer oder einer Kombination aus mindestens zwei davon. Ein bevorzugter steroidaler Entzündungshemmer ist einer ausgewählt aus der Gruppe bestehend aus Dexamethason, Hydrocortison und Prednisolon oder eine Kombination aus mindestens zwei davon. Ein bevorzugter nichtsteroidaler Entzündungshemmer ist einer ausgewählt aus der Gruppe bestehend aus Ibuprofen, Acetylsalicylsäure, Diclofenac, Indometacin und Phenylbutazon oder eine Kombination aus mindestens zwei davon. Ein bevorzugter pflanzlicher Entzündungshemmer ist ein ätherisches Öl. Ein bevorzugtes ätherisches Öl beinhaltet Bestandteile, die aus einer Kamillenblüte oder einer Arnikablüte oder aus beiden gewonnen wurden. Ein besonders bevorzugter Entzündungshemmer ist einer steroidaler Entzündungshemmer. Ein bevorzugter Hohlraum, der zu dem Umgebungsvolumen hin offen ist, ist eine Bohrung. Eine besonders bevorzugte Elektrode beinhaltet eine Pluralität von Hohlräumen, wobei die Hohlräume zu dem Umgebungsvolumen hin offen sind, und jeweils einen Wirkstoff beinhalteten.

In einer erfindungsgemäßen Ausführungsform ist der Hohlkörper ein Therapiegerät. Ein bevorzugtes Therapiegerät ist implantierbar. Ein bevorzugtes implantierbares Therapiegerät ist in ein Herz implantierbar. Ein weiteres bevorzugtes Therapiegerät ist ein Defibrillator oder ein Schrittmacher oder eine Kombination aus mindestens zwei davon. Ein weiteres bevorzugtes Therapiegerät ist ein implantierbarer Defibrillator oder ein implantierbarer Schrittmacher oder beides. Ein bevorzugter Schrittmacher ist einer ausgewählt aus der Gruppe bestehend aus einem Herzschrittmacher, einem Blasenschrittmacher, einem Darmschrittmacher, einem Hirnschrittmacher, einem Atemschrittmacher und einem Zwerchfellschrittmacher oder eine Kombination aus mindestens zwei davon. Ein besonders bevorzugter Schrittmacher ist ein Herzschrittmacher. Ein bevorzugter Herzschrittmacher ist ein kabelloser Herzschrittmacher. Ein weiteres bevorzugtes Therapiegerät beinhaltet keine flexible Elektrodenleitung, insbesondere keine flexible Elektrodenleitung zwischen einem Gehäuse und einer Elektrode. Ein weiteres bevorzugtes Therapiegerät ist ein Herztherapiegerät, bevorzugt ein Herzschrittmacher, bevorzugter ein kabelloser Herzschrittmacher.

In einer weiteren erfindungsgemäßen Ausführungsform ist der Hohlkörper ein Diagnosegerät. Ein bevorzugtes Diagnosegerät ist implantierbar. Ein weiteres bevorzugtes Diagnosegerät ist ein Biomonitor. Ein bevorzugter Biomonitor beinhaltet eines ausgewählt aus der Gruppe bestehend aus einem EKG-Gerät, einem Holter-Monitor, einem Event-Recorder, und einem Loop-Recorder, oder eine Kombination von mindestens zwei davon. Ein bevorzugtes EKG-Gerät ist ein Langzeit-EKG-Gerät, welches bevorzugt Daten speichert, die über einen Zeitraum von mindestens einer Stunde anfallen. Ein weiteres bevorzugtes Diagnosegerät beinhaltet eine Sendeeinrichtung oder einen Datenspeicher oder beides. Eine bevorzugte Sendeeinrichtung ist ausgebildet zu einem drahtlosen, bevorzugt telemetrischen, Übertragen von Daten, bevorzugt EKG-Daten. Ein bevorzugtes drahtloses Übertragen von Daten ist ein Übertragen mittels Wellen. Bevorzugte Wellen sind Longitudinalwellen oder Transversalwellen oder beides. Bevorzugte Longitudinalwellen sind akustische Wellen oder Schallwellen oder beides. Bevorzugte Transversalwellen sind elektromagnetische Wellen. Bevorzugte elektromagnetische Wellen sind Wellen der Frequenz eines Mobilfunknetzes oder von Bluetooth oder beides. Ein bevorzugtes Mobilfunknetz ist ein GSM-Netz. Als Datenspeicher kann jede Einheit zum Speichern von Daten ausgewählt werden, welche der Fachmann für geeignet zum Speichern medizinischer Daten, bevorzugt EKG-Daten, in einem implantierbaren Gerät hält. Ein bevorzugter Datenspeicher ist ein Magnetspeicher oder ein Flashspeicher oder beides.

In einer erfindungsgemäßen Ausführungsform ist das erste Bauteil ein Gehäuse; und beinhaltet das erste Bauteil einen Anschlussflansch, beinhaltend eine Flanschöffnung, wobei die Flanschöffnung das zweite Bauteil beinhaltet; wobei das zweite Bauteil ein Rahmen, beinhaltend eine Rahmenöffnung, ist; wobei die Elektrode die Rahmenöffnung durchstößt. Der Anschlussflansch ist bevorzugt in eine Öffnung des Gehäuses eingepasst und angeschweißt. Bevorzugt ist der Rahmen in die Flanschöffnung eingepasst und angelötet. Weiter bevorzugt wurden der Rahmen und die Elektrode einstückig in einem Sinterprozess hergestellt.

In einer erfindungsgemäßen Ausführungsform ist das erste Bauteil ein Gehäuse; und beinhaltet das Gehäuse eine Gehäuseöffnung, wobei die Gehäuseöffnung das zweite Bauteil beinhaltet; wobei das zweite Bauteil ein Rahmen, beinhaltend eine Rahmenöffnung, ist; wobei die Elektrode die Rahmenöffnung durchstößt; wobei der Rahmen einen Metallgehalt beinhaltet; wobei der Metallgehalt des Rahmens radial nach außen zunimmt. Der Rahmen ist bevorzugt in die Gehäuseöffnung eingepasst und angeschweißt. Weiter bevorzugt wurden der Rahmen und die Elektrode einstückig in einem Sinterprozess hergestellt. Ein bevorzugter Metallgehalt weist mindestens zwei, bevorzugt mindestens drei, bevorzugter mindestens vier, am bevorzugtesten mindestens fünf, Sprünge entlang eines Radius des Rahmens auf. Ein weiterer bevorzugter Metallgehalt ist eine stetige Funktion einer Position auf einem Radius des Rahmens. Ein bevorzugter Metallgehalt beinhaltet einen Gradienten des Metallgehalts in einem Bereich von 20 bis 80 Gew.-% $\times$ mm$^{-1}$, bevorzugt in einem Bereich von 25 bis 75 Gew.-% $\times$ mm$^{-1}$, bevorzugter in einem Bereich von 30 bis 70 Gew.-% $\times$ mm$^{-1}$, am bevorzugtesten in einem Bereich von 35 bis 65 Gew.-% $\times$ mm$^{-1}$, entlang eines Radius des Rahmens, jeweils bezogen auf das Gesamtgewicht des Rahmens.

Ein bevorzugter Rahmen beinhaltet einen ersten, einen zweiten und einen drittem konzentrischen Teilrahmen, wobei ein Metallgehalt des ersten Teilrahmens in einem Bereich von 0 bis 60 Gew.-%, bevorzugt in einem Bereich von 10 bis 50 Gew.-%, bevorzugter in einem Bereich von 20 bis 40 Gew.-%, bezogen auf das Gewicht des ersten Teilrahmen ist; wobei ein Metallgehalt des zweiten Teilrahmens in einem Bereich von 20 bis 80 Gew.-%, bevorzugt in einem Bereich von 30 bis 70 Gew.-%, bevorzugter in einem Bereich von 40 bis 60 Gew.-%, bezogen auf das Gewicht des zweiten Teilrahmen ist; wobei ein Metallgehalt des dritten Teilrahmens in einem Bereich von 40 bis 100 Gew.-%, bevorzugt in einem Bereich von 50 bis 90 Gew.-%, bevorzugter in einem Bereich von 60 bis 80 Gew.-%, bezogen auf das Gewicht des dritten Teilrahmen ist.

Die Herstellung eines erfindungsgemäßen Rahmens, beinhaltend einen Metallgehalt, wobei der Metallgehalt des Rahmens radial nach außen zunimmt, kann wie folgt realisiert werden. In der Gehäuseöffnung können eine oder mehrere organische Folien mit niedrigem Brennpunkt konzentrisch angeordnet werden. Dabei trennen die Folien verschiedene konzentrische Volumina voneinander ab. In diese Volumina können dann Pulver ausgewählt aus der Gruppe bestehend aus einem Metall-Pulver, einem Keramik-Pulver und einem Cermet-Pulver oder eine Kombination aus mindestens zwei davon eingebracht werden. Dabei werden die Pulver so eingebracht, dass der in die verschiedenen Volumina eingebrachte Metallgehalt von innen liegenden Volumina zu außen liegenden Volumina zunimmt. Durch die organischen Folien abgetrennt mischen sich die in die verschiedenen Volumina eingebrachten Materialien während des Sinterns nicht. Da die organischen Folien eine Brenntemperatur haben, die unterhalb der Sintertemperatur liegt, lösen die Folien sich beim Sintern auf und diffundieren aus dem Rahmen heraus. Nachdem die organischen Folien sich aufgelöst haben, berühren sich die aneinandergrenzenden Materialien der zunächst separat befüllten Volumina und gehen ihrerseits jeweils eine stoffschlüssige gesinterte Verbindung ein.

In einer erfindungsgemäßen Ausführungsform ist das zweite Bauteil ein Gehäuse. Ein bevorzugtes Gehäuse ist längserstreckt.

Einen Beitrag zur Erfüllung mindestens einer der erfindungsgemäßen Aufgaben leistet ein Verfahren zu einer Herstellung einer Vorrichtung, beinhaltend als Verfahrensschritte

a) Bereitstellen eines Gehäuses, beinhaltend eine Gehäuseöffnung,
b) Einbringen eines elektronischen Bauteils in das Gehäuse durch die Gehäuseöffnung;
c) Verschließen der Gehäuseöffnung mit einem Verschluss;
d) Verbinden des Gehäuses mit einer Elektrode;

wobei die Elektrode ein Cermet beinhaltet, wobei die Elektrode das elektronische Bauteil elektrisch leitend mit einer Umgebung des Gehäuses verbindet, wobei die Elektrode eine Kontaktoberfläche beinhaltet, wobei die Kontaktoberfläche ausgebildet ist zu einem Kontaktieren eines eukaryotisch Gewebes, wobei die Kontaktoberfläche einen maximalen Abstand zu dem elektronischen Bauteil von weniger als 80 mm, bevorzugt weniger als 70 mm, bevorzugter weniger als 60 mm, bevorzugter weniger als 50 mm, bevorzugter weniger als 45 mm, bevorzugter weniger als 40 mm, bevorzugter weniger als 35 mm, bevorzugter weniger als 30 mm, bevorzugter weniger als 25 mm, bevorzugter weniger als 20 mm, bevorzugter weniger als 15 mm, noch bevorzugter weniger als 10 mm, am bevorzugtesten weniger als 5 mm, hat. Ein bevorzugtes elektronisches Bauteil ist ein elektronisches Bauteil gemäß der vorstehend beschriebenen erfindungsgemäßen Vorrichtung. Bevorzugt beinhaltet das Verfahren einen weiteren Verfahrensschritt, bei dem eine offene Porendichte der Kontaktoberfläche oder einer Teiloberfläche der Kontaktoberfläche oder beider durch ein Ätzen der entsprechenden Oberfläche vergrößert wird. Bei einem bevorzugten Ätzen wird ein keramischer Anteil des Cermets weggeätzt, was zu einer Porenbildung führt, und ein metallischer Anteil des Cermets bleibt bestehen.

Bevorzugt verbindet die Elektrode das elektronische Bauteil elektrisch leitend mit der Umgebung des Gehäuses über einen ohmschen Widerstand von nicht mehr als 10 $\Omega$, bevorzugt nicht mehr als 1 $\Omega$, bevorzugter nicht mehr als 200 m$\Omega$, noch bevorzugter nicht mehr als 100 m$\Omega$, am bevorzugtesten nicht mehr 50 m$\Omega$.

Ein bevorzugtes Gehäuse ist elektrisch leitend ausgebildet. Ein weiteres bevorzugtes Gehäuse beinhaltet ein Metall. Ein weiteres bevorzugtes Gehäuse beinhaltet ein biokompatibles Material. Ein weiteres bevorzugtes Gehäuse ist längserstreckt. Ein bevorzugter Verschluss beinhaltet ein biokompatibles Material. Ein bevorzugtes elektronisches Bauteil beinhaltet weiter eines ausgewählt aus der Gruppe bestehend aus einer Steuereinheit, einer Messeinheit und einer Batterie oder eine Kombination aus mindestens zwei davon.

In einer erfindungsgemäßen Ausführungsform ist das Gehäuse elektrisch leitfähig;

wobei das Verbinden des Gehäuses mit der Elektrode als Unterschritte beinhaltet

a) Verbinden des Gehäuses mit einem Anschlussflansch, beinhaltend eine Flanschöffnung;
b) Einbringen eines Rahmens, beinhaltend eine Keramik und eine Rahmenöffnung, in die Flanschöffnung;

wobei die Elektrode die Rahmenöffnung durchstößt. Ein bevorzugtes Verbinden des Gehäuses mit dem Anschlussflansch ist ein Schweißen. Bevorzugt wird der Rahmen in die Flanschöffnung eingelötet. Weiter Bevorzugt wird der Rahmen in die Flanschöffnung mit einem Gold-Lot eingelötet. Weiter bevorzugt wurden der Rahmen und die Elektrode einstückig in einem Sinterprozess hergestellt.

In einer erfindungsgemäßen Ausführungsform ist das Gehäuse elektrisch leitfähig; wobei das Verbinden des Gehäuses mit der Elektrode als Unterschritt beinhaltet ein Verbinden des Gehäuses mit einem Rahmen, beinhaltend eine Rahmenöffnung, eine Keramik und einen Metallgehalt; wobei die Elektrode die Rahmenöffnung durchstößt, wobei der Metallgehalt des Rahmens radial nach außen zunimmt. Ein bevorzugter Metallgehalt weist mindestens zwei, bevorzugt mindestens drei, bevorzugter mindestens vier, am bevorzugtesten mindestens fünf, Sprünge entlang eines Radius des Rahmens auf. Ein weiterer bevorzugter Metallgehalt ist eine stetige Funktion entlang einer Position auf einem Radius des Rahmens. Ein bevorzugter Metallgehalt beinhaltet einen Gradienten des Metallgehalts in einem Bereich von 20 bis 80 Gew.-% $\times$ mm$^{-1}$, bevorzugt in einem Bereich von 25 bis 75 Gew.-% $\times$ mm$^{-1}$, bevorzugter in einem Bereich von 30 bis 70 Gew.-% $\times$ mm$^{-1}$, am bevorzugtesten in einem Bereich von 35 bis 65 Gew.-% $\times$ mm$^{-1}$, entlang eines Radius des Rahmens, jeweils bezogen auf das Gesamtgewicht des Rahmens.

Ein bevorzugter Rahmen beinhaltet einen ersten, einen zweiten und einen drittem konzentrischen Teilrahmen, wobei ein Metallgehalt des ersten Teilrahmens in einem Bereich von 0 bis 60 Gew.-%, bevorzugt in einem Bereich von 10 bis 50 Gew.-%, bevorzugter in einem Bereich von 20 bis 40 Gew.-%, bezogen auf das Gewicht des ersten Teilrahmen ist; wobei ein Metallgehalt des zweiten Teilrahmens in einem Bereich von 20 bis 80 Gew.-%, bevorzugt in einem Bereich von 30 bis 70 Gew.-%, bevorzugter in einem Bereich von 40 bis 60 Gew.-%, bezogen auf das Gewicht des zweiten Teilrahmen ist; wobei ein Metallgehalt des dritten Teilrahmens in einem Bereich von 40 bis 100 Gew.-%, bevorzugt in einem Bereich von 50 bis 90 Gew.-%, bevorzugter in einem Bereich von 60 bis 80 Gew.-%, bezogen auf das Gewicht des dritten Teilrahmen ist.

Ein bevorzugtes Verbinden des Gehäuses mit dem Rahmen ist ein Schweißen oder ein Löten oder beides. Ein bevorzugtes Löten ist ein Löten mit einem Gold-Lot. Weiter bevorzugt wurden der Rahmen und die Elektrode einstückig in einem Sinterprozess hergestellt.

In einer erfindungsgemäßen Ausführungsform beinhaltet das Gehäuse eine Keramik. Ein bevorzugtes Gehäuse ist elektrisch isolierend.

In einer erfindungsgemäßen Ausführungsform ist der Verschluss elektrisch leitfähig. Ein bevorzugter Verschluss beinhaltet ein Metall. Ein bevorzugter Verschluss ist hermetisch dicht. Ein weiterer bevorzugter Verschluss ist verschweißt oder verlötet oder beides. Ein weiterer bevorzugter Verschluss ist mit einem Gold-Lot verlötet.

Einen Beitrag zur Erfüllung mindestens einer der erfindungsgemäßen Aufgaben leistet eine Vorrichtung erhältlich durch das erfindungsgemäße Verfahren.

Einen Beitrag zur Erfüllung mindestens einer der erfindungsgemäßen Aufgaben leistet eine Verwendung einer Elektrode zu einem Herstellen eines kabellosen Herzschrittmachers, wobei die Elektrode ein Cermet beinhaltet. Eine bevorzugte Elektrode beinhaltet ein Cermet, welches ein Metall zu mindestens 25 Vol.-%, bevorzugt zu mindestens 32 Vol.-%, am bevorzugtesten zu mindestens 38 Vol.-%, basierend auf dem Volumen der Elektrode beinhaltet.

Einen Beitrag zur Erfüllung mindestens einer der erfindungsgemäßen Aufgaben leistet ein Verfahren beinhaltend als Verfahrensschritte

a) Bereitstellen einer erfindungsgemäßen Vorrichtung,
b) Kontaktieren eines eukaryotischen Gewebes mit der Kontaktoberfläche.

In einer erfindungsgemäßen Ausführungsform wird in einem weiteren Verfahrensschritt der Hohlkörper in einen eukaryotischen Organismus eingebracht wird. Ein bevorzugtes Einbringen des Hohlkörpers in den eukaryotischen Organismus ist ein Implantieren.

Einen Beitrag zur Erfüllung mindestens einer der erfindungsgemäßen Aufgaben leistet eine Verwendung einer erfindungsgemäßen Vorrichtung zu einer Therapie von Bradykardie.

erstes Bauteil

Ein bevorzugtes erstes Bauteil ist eines ausgewählt aus der Gruppe bestehend aus einem Gehäuse, einem Verschluss, einem Rahmen, einer Platte oder eine Kombination von mindestens zwei davon. Ein besonders bevorzugtes erstes Bauteil ist eine Anode. Ein weiteres bevorzugtes erstes Bauteil beinhaltet ein Metall. Ein weiteres bevorzugtes erstes Bauteil beinhaltet ein biokompatibles Material.

zweites Bauteil

Ein bevorzugtes zweites Bauteil ist ein Rahmen oder ein Gehäuse oder beides. Ein weiteres bevorzugtes zweites Bauteil ist elektrisch isolierend. Ein weiteres bevorzugtes zweites Bauteil beinhaltet eine Keramik. Ein besonders bevorzugtes zweites Bauteil besteht aus einer Keramik.

Rahmen

Ein Rahmen ist erfindungsgemäß bevorzugt ein Torus, torusförmig, oder ein Prisma, wobei das Prisma eine erste Grundfläche und eine zweite Grundfläche und mindestens einen Hohlraum beinhaltet, wobei der Hohlraum eine Teilfläche der ersten Grundfläche und eine Teilfläche der zweiten Grundfläche beinhaltet. Der Hohlraum wird als Rahmenöffnung bezeichnet. Eine bevorzugte erste Grundfläche oder eine bevorzugte zweite Grundfläche oder beide sind eines ausgewählt aus der Gruppe bestehend aus einer Kreisfläche, einer Ellipsenfläche, einer Ovalfläche, einer Dreiecksfläche, einer Viereckfläche, einer Fünfecksfläche, einer Sechsecksfläche, einer Siebenecksfläche, einer Achtecksfläche, einer Polygonfläche oder eine Kombination von mindestens zwei davon. Die Teilfläche der ersten Grundfläche oder die Teilfläche der zweiten Grundfläche oder beide, die die Rahmenöffnung beinhaltet sind bevorzugt eines ausgewählt aus der Gruppe bestehend aus einer Kreisfläche, einer Ellipsenfläche, einer Ovalfläche, einer Dreiecksfläche, einer Viereckfläche, einer Fünfecksfläche, einer Sechsecksfläche, einer Siebenecksfläche, einer Achtecksfläche, einer Polygonfläche oder eine Kombination von mindestens zwei davon. Eine bevorzugte Polygonfläche ist eine Fläche eines regelmäßigen Polygons oder eine Fläche eines unregelmäßigen Polygons. Ein ganz bevorzugter Rahmen ist ein Hohlzylinder oder ein Ring oder beides. Ein weiterhin bevorzugter Rahmen ist eine Lochplatte. Eine Lochplatte ist eine Platte, beinhaltend eine Pluralität von sich gegenüberliegenden Flächen verbindenden Löchern. Ein Radius des Rahmens ist für die Verwendung in diesem Dokument wie folgt definiert. Ist der Rahmen ein Torus oder torusförmig, so ist der Radius des Rahmens der große Radius des Torus. Ist der Rahmen ein Prisma, so ist der Radius des Rahmens eine Gerade, welche einen Flächenschwerpunkt einer Grundfläche des Prismas mit einem Punkt auf dem Umfang der Grundfläche verbindet. Ist der Rahmen eine Lochplatte, so ist der Radius des Rahmes eine Gerade, welche einen Flächenschwerpunkt einer Grundfläche der Lochplatte mit einem Punkt auf dem Umfang der Lochplatte verbindet.

Kontaktoberfläche

Eine erfindungsgemäße Kontaktoberfläche ist eine Oberfläche der Elektrode, welche das Umgebungsvolumen berührt. Eine bevorzugte Kontaktoberfläche ist ausgebildet, um eine möglichst große äußere Oberfläche zu beinhalten. Eine bevorzugte Kontaktoberfläche beinhaltet eine geometrische Form mit einer möglichst großen äußeren Oberfläche. Eine weitere bevorzugte Kontaktoberfläche ist zu dem Umgebungsvolumen hin ausgewölbt. Eine weitere bevorzugte Kontaktoberfläche ist gesintert.

elektrischer Impulsgenerator

Ein erfindungsgemäßer elektrischer Impulsgenerator ist eine elektronische Schaltung oder ein elektronisches Gerät oder beides, welche oder welches ausgebildet ist um einmalig oder wiederholt für einen kurzen Zeitraum einen elektrischen Spannungspuls abzugeben. Ein bevorzugter elektrischer Spannungspuls ist ein Gleichspannungspuls. Ein weiterer bevorzugter Spannungspuls hat einen maximalen Spannunsgwert von weniger als 24 V, bevorzugt weniger als 12 V, bevorzugter weniger als 10 V, am bevorzugtesten weniger als 2,4 V. Ein bevorzugter kurzer Zeitraum ist kürzer als 500 ms, bevorzugt kürzer als 100 ms, bevorzugter kürzer als 50 ms, am bevorzugtesten kürzer als 10 ms.

Elektrode

Eine bevorzugte Elektrode ist eine Kathode oder eine Anode oder beides. Eine besonders bevorzugte Elektrode ist eine Kathode. Eine weitere bevorzugte Elektrode ist eine Tippelektrode oder eine Ringelektrode oder beides. Eine besonders bevorzugte Elektrode ist eine Tippelektrode. Eine ganz besonders bevorzugte Elektrode ist eine Tippkathode. Ein erfindungsgemäß bevorzugter Hohlkörper beinhaltet mindestens zwei Elektroden, von denen mindestens eine erfindungsgemäß ausgestaltet ist. Eine weitere bevorzugte Elektrode besteht aus einem Cermet.

Metall

Hier kommen alle dem Fachmann geläufigen Metalle in Betracht, die neben einer Leitfähigkeit auch eine gute Verträglichkeit mit eukaryotischem Gewebe aufweisen. Ein erfindungsgemäß bevorzugtes Metall ist vorzugsweise ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder eine Kombination von mindestens zwei davon. Dabei ist eine bevorzugte Kombination eine Legierung. Ein bevorzugter Edelstahl ist ein Edelstahl 316L. Ein bevorzugtes Metall ist biokompatibel. Eine bevorzugte Legierung ist biokompatibel.

Cermet

Erfindungsgemäß wird als "Cermet" ein Verbundwerkstoff aus einem oder mehreren keramischen Werkstoffen in mindestens einer metallischen Matrix oder ein Verbundwerkstoff aus einem oder mehreren metallischen Werkstoffen in mindestens einer keramischen Matrix oder beides bezeichnet. Zur Herstellung eines Cermets kann beispielsweise ein Gemisch aus mindestens einem keramischen Pulver und mindestens einem metallischen Pulver verwendet werden, welches beispielsweise mindestens mit einem Bindemittel und ggf. mindestens mit einem Lösungsmittel versetzt werden kann. Das bzw. die keramischen Pulver des Cermets weisen vorzugsweise eine mittlere Korngröße von weniger als 10 $\mu$m, bevorzugt weniger als 5 $\mu$m, besonders bevorzugt weniger als 3 $\mu$m auf. Das bzw. die metallischen Pulver des Cermets wiesen vorzugsweise eine mittlere Korngröße von weniger als 15 $\mu$m, bevorzugt weniger als 10 $\mu$m, besonders bevorzugt weniger als 5 $\mu$m auf. Als mittlere Korngröße wird dabei insbesondere der Medianwert oder $D_{50}$-Wert der Korngrößenverteilung angesehen. Der $D_{50}$-Wert beschreibt jenen Wert, bei dem 50 % der Körner des keramischen Pulvers und/oder des metallischen Pulvers feiner sind als der $D_{50}$-Wert. Ein bevorzugtes Cermet weist eine hohe spezifische Leitfähigkeit auf, die bevorzugt mindestens 1 S/m, bevorzugter mindestens 100 S/m, bevorzugter mindestens $10^3$ S/m, bevorzugter mindestens $10^4$ S/m , noch bevorzugter mindestens $10^5$ S/m, und am bevorzugtesten mindestens $10^6$ S/m beträgt.

Die mindestens eine keramische Komponente eines erfindungsgemäßen Cermets beinhaltet bevorzugt eine erfindungsgemäße Keramik. Die mindestens eine metallische Komponente eines erfindungsgemäßen Cermets beinhaltet bevorzugt eines ausgewählt aus der Gruppe bestehend aus Platin, Iridium, Niob, Palladium, Eisen, Edelstahl, Kobald-Chrom-Legierung, Molybdän, Tantal, Wolfram, Titan, Kobalt und Zirkonium oder Kombination von mindestens zwei davon. Dabei ist eine bevorzugte Kombination eine Legierung. Ein bevorzugter Edelstahl ist ein Edelstahl 316L. Eine elektrisch leitfähige Verbindung stellt sich im Cermet in der Regel dann ein, wenn der Metallgehalt über der sogenannten Perkolationsschwelle liegt, bei der die Metallpartikel im gesinterten Cermet mindestens punktuell miteinander verbunden sind, so dass eine elektrische Leitung ermöglicht wird. Dazu sollte der Metallgehalt erfahrungsgemäß, abhängig von der Materialauswahl, mindestens 25 Vol.-% betragen, bevorzugt mindestens 32 Vol.-%, am bevorzugtesten mindestens 38 Vol.-%, jeweils bezogen auf das gesamte Volumen des Cermets.

Keramik

Eine erfindungsgemäße Keramik kann jede Keramik sein, die der Fachmann für den erfindungsgemäßen Einsatz auswählen würde. Die Keramik ist bevorzugt ausgewählt aus der Gruppe bestehend aus einer Oxidkeramik, einer Silikatkeramik, einer Nichtoxid-Keramik oder einer Mischung aus mindestens zwei davon.

Die Oxidkeramik ist bevorzugt ausgewählt aus der Gruppe bestehend aus einem Metalloxid, einem Halbmetalloxid oder einer Mischung davon. Das Metall des Metalloxids kann ausgewählt sein aus der Gruppe bestehend aus Aluminium, Beryllium, Barium, Calcium, Magnesium, Natrium, Kalium, Eisen, Zirkonium, Titan oder einer Mischung von mindestens zwei davon. Das Metalloxid ist bevorzugt ausgewählt aus der Gruppe bestehend aus Aluminiumoxid ($Al_2O_3$), Magnesiumoxid (MgO), Zirkoniumoxid ($ZrO_2$), Yttriumoxid ($Y_2O_3$), Aluminiumtitanat ($Al_2TiO_5$), einer Piezokeramik wie Blei-Zirkonat ($PbZrO_3$), Blei-Titanat ($PbTiO_3$) sowie Blei-Zirkonat-Titanat (PZT) oder einer Mischung von mindestens zwei hiervon. Das Halbmetall des Halbmetalloxids ist bevorzugt ausgewählt aus der Gruppe bestehend aus Bor, Silicium, Arsen, Tellur oder einer Mischung von mindestens zwei davon. Eine weitere bevorzugte Oxidkeramik beinhaltet eines ausgewählt aus der Gruppe bestehend aus Zirkoniumoxid verstärktes Aluminiumoxid (ZTA - Zirconia Toughened Aluminum - $Al_2O_3/ZrO_2$), Yttrium verstärktes Zirkoniumoxid (Y-TZP), Barium(Zr, Ti)oxid, Barium(Ce, Ti)oxid oder eine Kombination aus mindestens zwei davon.

Die Silikatkeramik ist bevorzugt ausgewählt aus der Gruppe bestehend aus einem Steatit ($Mg_3[Si_4O_{10}(OH)_2]$), Cordierit ($(Mg, Fe^{2+})_2(Al_2Si)[Al_2Si_4O_{18}]$), Mullit ($Al_2Al_{2+2x}Si_{2-2x}O_{10-x}$ mit x = Sauerstoffleerstellen pro Elementarzelle), Feldspat ($(Ba,Ca,Na,K,NH_4)(Al,B,Si)_4O_8$) oder einer Mischung aus mindestens zwei davon.

Die Nichtoxid-Keramik kann ausgewählt sein aus der Gruppe bestehend aus einem Carbid, einem Nitrid oder einer Mischung daraus. Das Carbid kann ausgewählt sein aus der Gruppe bestehend aus Siliciumcarbid (SiC), Borcarbid ($B_4C$), Titancarbid (TiC), Wolframcarbid, Zementit (Fe3C). Das Nitrid kann ausgewählt sein aus der Gruppe bestehend aus Siliciumnitrid ($Si_3N_4$), Aluminiumnitrid (AlN), Titannitrid (TiN), Siliciumaluminiumoxinitrid (SIALON) oder einer Mischung aus mindestens zwei davon. Eine weitere bevorzugte Nichtoxid-Keramik ist Natrium-Kalium-Niobat.

biokompatibles Material

Ein bevorzugtes biokompatibles Material ist eines ausgewählt aus der Gruppe bestehend aus biotolerant, bioinert und bioaktiv oder eine Kombination aus mindestens zwei davon.

Eukaryotisches Gewebe

Ein bevorzugtes eukaryotisches Gewebe ist ein tierisches Gewebe oder ein menschliches Gewebe oder beides.

Abstand

Erfindungsgemäß ist ein Abstand zwischen der Kontaktoberfläche und dem elektronischen Bauteil eine Länge einer kürzesten Geraden, welche einen Punkt auf der Kontaktoberfläche und einen Punkt auf einer Oberfläche des elektronischen Bauteils verbindet. Ein maximaler Abstand zwischen der Kontaktoberfläche und dem elektronischen Bauteil ist ein Abstand, bei dem der Punkt auf der Kontaktoberfläche und der Punkt auf einer Oberfläche des elektronischen Bauteils so gewählt wurden, dass der Abstand maximal wird.

elektrisch leitend

Ein Körper ist für die Verwendung in diesem Dokument elektrisch leitend, wenn eine elektrische Leitfähigkeit des Körpers mindestens 0,01 S/m ist. Ein Körper ist elektrisch isolierend, wenn der Körper nicht elektrisch leitend ist. Eine Oberfläche oder Teiloberfläche ist für die Verwendung in diesem Dokument elektrisch leitend; wenn ein gerades Prisma, welches die Oberfläche oder die Teiloberfläche als eine Grundfläche beinhaltet, eine identische Fläche als die zweite Grundfläche beinhaltet, eine Höhe von 1 m hat, und vollständig aus dem Material der Oberfläche oder Teiloberfläche besteht; eine elektrische Leitfähigkeit von mindestens 0,01 S/m besitzt. Eine Fläche ist elektrisch isolierend, wenn die Fläche nicht elektrisch leitend ist.

innere Oberfläche

Für die Verwendung in diesem Dokument ist eine innere Oberfläche einer Fläche ein Flächeninhalt der Fläche einschließlich aller Flächeninhalte aller Poren, welche zu der Fläche offen sind.

äußere Oberfläche

Für die Verwendung in diesem Dokument ist eine äußere Oberfläche einer Fläche ein Flächeninhalt einer von außen mit dem menschlichen Auge sichtbaren Teilfläche der Fläche. Insbesondere ist eine äußere Oberfläche einer Fläche ein Flächeninhalt einer Einhüllenden der Fläche, wobei die Einhüllende alle offenen Poren der Fläche überdeckt und sich nicht in die offenen Poren hineinlegt.

hermetisch dicht

Der Hohlkörper trennt bevorzugt das Innenvolumen hermetisch dicht von dem Umgebungsvolumen. Im Rahmen der Erfindung kann der Begriff "hermetisch dicht" dabei verdeutlichen, dass bei einem bestimmungsgemäßen Gebrauch innerhalb üblicher Zeiträume (beispielsweise 5 bis 10 Jahre) Feuchtigkeit oder Gase oder beides nicht oder nur minimal durch den hermetisch dichten Hohlkörper hindurch zwischen Umgebungsvolumen und Innenvolumen ausgetauscht werden können. Eine physikalische Größe, die beispielsweise eine Permeation von Gasen oder Feuchtigkeit oder beidem durch den Hohlkörper beschreiben kann, ist die sogenannte Leckrate, welche beispielsweise durch Lecktests bestimmt werden kann. Entsprechende Lecktests können beispielsweise mit Heliumlecktestern und/oder Massenspektrometern durchgeführt werden und sind im Standard Mil-STD-883G Method 1014 spezifiziert. Die maximal zulässige Helium-Leckrate wird dabei abhängig vom internen Volumen der zu prüfenden Vorrichtung, hier dem Innenvolumen, festgelegt. Nach den in MIL-STD-883G, Method 1014, in Absatz 3.1 spezifizierten Methoden, und unter Berücksichtigung der in der Anwendung der vorliegenden Erfindung vorkommenden Volumina und Kavitäten der zu prüfenden Vorrichtungen, können diese maximal zulässigen Helium-Leckraten beispielsweise von $1 \times 10^{-8}$ atm$\times$cm$^3$/s bis $1 \times 10^{-7}$ atm$\times$cm$^3$/s betragen. Im Rahmen der Erfindung kann der Begriff "hermetisch dicht" insbesondere bedeuten, dass der Hohlkörper eine Helium-Leckrate von weniger als $1 \times 10^{-7}$ atm$\times$cm$^3$/s aufweist. In einer vorteilhaften Ausführung kann die Helium-Leckrate weniger als $1 \times 10^{-8}$ atm$\times$cm$^3$/s, insbesondere weniger als $1 \times 10^{-9}$ atmcm$^3$/s betragen.

Zum Zweck der Standardisierung können die genannten Helium-Leckraten auch in die äquivalente Standard-Luft-Leckrate konvertiert werden. Die Definition für die äquivalente Standard-Luft-Leckrate (Equivalent Standard Air Leak Rate) und die Umrechnung sind im Standard ISO 3530 angegeben. Aufgrund der Einsatzart von implantierbaren Therapiegeräten ist deren hermetische Dichtigkeit und Biokompatibilität in der Regel eine der vorrangigsten Anforderungen. Der hier vorgeschlagene Hohlkörper kann insbesondere in einen Körper eines menschlichen oder tierischen Benutzers, insbesondere eines Patienten, eingesetzt werden. Dadurch ist der Hohlkörper in der Regel einer Flüssigkeit eines Körpergewebes des Körpers ausgesetzt. Somit ist es in der Regel von Bedeutung, dass weder Körperflüssigkeit in den Hohlkörper eindringt, noch das Flüssigkeiten aus dem Hohlkörper austreten. Um dieses sicherzustellen, sollte der Hohlkörper eine möglichst vollständige Undurchlässigkeit aufweisen, insbesondere gegenüber Körperflüssigkeiten.

Anschlussflansch

Vorliegend ist es bevorzugt, dass der Anschlussflansch sich zum Verbinden eines keramischen Bauteils mit einer Öffnung eines metallischen Bauteils eignet. Ein erfindungsgemäßer Anschlussflansch beinhaltet bevorzugt ausschließlich Materialien, welche eines ausgewählt aus der Gruppe bestehend aus biokompatibel, leicht verarbeitbar, korrosionsbeständig und dauerhaft stoffschlüssig mit dem ersten Element und dem zweiten Element verbindbar oder eine Kombination aus mindestens zwei davon sind. Ein bevorzugter Anschlussflansch beinhaltet eines ausgewählt aus der Gruppe bestehend aus Platin, Iridium, Niob, Molybdän, Tantal, Wolfram, Titan, Kobalt-Chrom-Legierungen und Zirkonium oder eine Kombination aus mindestens zwei davon. Dabei ist eine bevorzugte Kombination eine Legierung.

Sintern

Unter einem Sintern oder einem Sinterprozess wird im Rahmen der vorliegenden Erfindung allgemein ein Verfahren zur Herstellung von Werkstoffen oder Werkstücken verstanden, bei welchem pulverförmige, insbesondere eines ausgewählt aus der Gruppe bestehend aus feinkörnige Stoffe, keramische Stoffe und metallische Stoffe oder eine Kombination aus mindestens zwei davon erhitzt werden und dadurch verbunden werden. Dieser Prozess kann ohne äu-ßeren Druck auf den zu erhitzenden Stoff erfolgen oder kann insbesondere unter erhöhtem Druck auf den zu erhitzenden Stoff erfolgen, beispielsweise unter einem Druck von mindestens 2 bar, vorzugsweise höheren Drucken, beispielsweise Drucken von mindestens 10 bar, insbesondere mindestens 100 bar oder sogar mindestens 1000 bar. Der Prozess kann insbesondere vollständig oder teilweise bei Temperaturen unterhalb der Schmelztemperatur der pulverförmigen Werkstoffe erfolgen, beispielsweise bei Temperaturen von 700°C bis 1400 °C. Der Prozess kann insbesondere vollständig oder teilweise in einem Werkzeug oder einer Form oder beides durchgeführt werden, so dass mit dem Sinterprozesses eine Formgebung verbunden werden kann. Neben den pulverförmigen Werkstoffen kann ein Ausgangsmaterial für den Sinterprozess weitere Werkstoff umfassen, beispielsweise einen oder mehrere Binder, oder ein oder mehrere Lösungsmittel, oder beides. Der Sinterprozess kann in einem Schritt oder auch in mehreren Schritten erfolgen, wobei dem Sinterprozess beispielsweise weitere Schritte vorgelagert sein können, beispielsweise ein oder mehrere Formgebungsschritte, oder ein oder mehrere Entbinderungsschritte, oder beides. Das Sintern bzw. der Sinterprozess entspricht somit einem Brennprozess. Der Sinterprozess, insbesondere für ein Cermet, kann vergleichbar zu einem üblicherweise für homogene Pulver verwendeten Sinterprozess ablaufen. Beispielsweise kann unter hoher Temperatur und ggf. hohem Druck das Material beim Sintervorgang verdichtet werden, so dass das Cermet nahezu dicht ist, oder eine höchstens geschlossene Porosität aufweist. Cermets zeichnen sich in der Regel durch eine besonders hohe Härte und Verschleißfestigkeit aus. Gegenüber Sinterhartmetallen hat eine Elektrode, beinhaltend ein Cermet, in der Regel eine höhere Thermoschock- und Oxidationsbeständigkeit und in der Regel einen an einen umgebenden Isolator angepassten thermischen Ausdehnungskoeffizienten.

Bevorzugte Ausführungsformen

Ausführungsform 1:

Vorrichtung beinhaltend einen Hohlkörper, ein Innenvolumen und ein Umgebungsvolumen; wobei

    a) das Innenvolumen ein elektronisches Bauteil beinhaltet;
    b) wobei der Hohlkörper

        i) das Innenvolumen umschließt,
        ii) ein erstes Bauteil, ein zweites Bauteil und eine Elektrode beinhaltet;

    c) das erste Bauteil elektrisch leitfähig ist;
    d) das zweite Bauteil die Elektrode elektrisch von dem ersten Bauteil isoliert;
    e) die Elektrode

        i) ein Cermet beinhaltet,
        ii) das Innenvolumen elektrisch leitend mit dem Umgebungsvolumen verbindet,
        iii) eine Kontaktoberfläche beinhaltet;

    f) die Kontaktoberfläche

        i) ausgebildet ist zu einem Kontaktieren eines eukaryotischen Gewebes,
        ii) einen maximalen Abstand zu dem elektronischen Bauteil von weniger als 80 mm hat.

Ausführungsform 2:

Die Vorrichtung nach Ausführungsform 1, wobei das elektronische Bauteil ein elektrischer Impulsgenerator ist.

Ausführungsform 3:

Die Vorrichtung nach Ausführungsform 1, wobei das elektronische Bauteil eines ausgewählt aus der Gruppe bestehend aus einem Datenspeicher, einer Datenverarbeitungseinheit, einer Energiequelle, einem Aufnahmegerät, und einer Sendeeinrichtung, oder eine Kombination von mindestens zwei davon ist.

Ausführungsform 4:

Die Vorrichtung nach einer der vorhergehenden Ausführungsformen, wobei die Elektrode als starrer Körper ausgebildet ist.

Ausführungsform 5:

Die Vorrichtung nach einer der vorhergehenden Ausführungsformen, wobei die Elektrode einstückig ausgebildet ist.

Ausführungsform 6:

Die Vorrichtung nach einer der vorhergehenden Ausführungsformen, wobei das Cermet ein Metall zu mindestens 25 Vol.-% basierend auf dem Volumen der Elektrode beinhaltet.

Ausführungsform 7:

Die Vorrichtung nach einer der vorhergehenden Ausführungsformen, wobei das zweite Bauteil eine Keramik beinhaltet.

Ausführungsform 8:

Die Vorrichtung nach einer der vorhergehenden Ausführungsformen, wobei der Hohlkörper das Innenvolumen hermetisch dicht von dem Umgebungsvolumen trennt.

Ausführungsform 9:

Die Vorrichtung nach einer der vorhergehenden Ausführungsformen, wobei der Hohlkörper ein Fixierelement beinhaltet, wobei das Fixierelement ausgebildet ist zu einem Fixieren einer Position des Hohlkörpers relativ zu einem eukaryotischen Gewebe.

Ausführungsform 10:

Die Vorrichtung nach einer der vorhergehenden Ausführungsformen, wobei der Hohlkörper entlang einer kartesischen Raumrichtung eine längste Ausdehnung besitzt, wobei die längste Ausdehnung weniger als 150 mm beträgt.

Ausführungsform 11:

Die Vorrichtung nach einer der vorhergehenden Ausführungsformen, wobei der Hohlkörper ein Volumen von höchstens 10 cm$^3$ einnimmt.

Ausführungsform 12:

Die Vorrichtung nach einer der vorhergehenden Ausführungsformen, wobei die Elektrode mindestens zwei Teilelektroden beinhaltet, wobei jede der Teilelektroden

a) ein Cermet beinhaltet,
b) das Innenvolumen elektrisch leitend mit dem Umgebungsvolumen verbindet,

c) von der jeweils anderen Teilelektrode oder den jeweils anderen Teilelektroden elektrisch isoliert ist.

Ausführungsform 13:

Die Vorrichtung nach einer der vorhergehenden Ausführungsformen, wobei die Kontaktoberfläche eine elektrisch leitende Teiloberfläche beinhaltet, wobei die elektrisch leitende Teiloberfläche eine äußere Oberfläche kleiner als 25 mm$^2$ hat.

Ausführungsform 14:

Die Vorrichtung nach einer der vorhergehenden Ausführungsformen, wobei die Kontaktoberfläche eine elektrisch leitende Teiloberfläche beinhaltet, wobei eine innere Oberfläche der elektrisch leitenden Teiloberfläche mindestens doppelt so groß ist wie die äußere Oberfläche der elektrisch leitenden Teiloberfläche.

Ausführungsform 15:

Die Vorrichtung nach einer der vorhergehenden Ausführungsformen, wobei die Kontaktoberfläche eine elektrisch isolierende Teiloberfläche beinhaltet, wobei die elektrisch isolierende Teiloberfläche eine äußere Oberfläche von mehr als 1 mm$^2$ hat.

Ausführungsform 16:

Die Vorrichtung nach einer der vorhergehenden Ausführungsformen, wobei die Kontaktoberfläche oder eine Teiloberfläche der Kontaktoberfläche oder beide eine mittlere Rauheit in einem Bereich von 0,2 bis 8 $\mu$m hat.

Ausführungsform 17:

Die Vorrichtung nach einer der vorhergehenden Ausführungsformen, wobei die Kontaktoberfläche oder eine Teiloberfläche der Kontaktoberfläche oder beide eine offene Porendichte in einem Bereich von 1000 bis 80000 ppi hat.

Ausführungsform 18:

Die Vorrichtung nach einer der vorhergehenden Ausführungsformen; wobei die Elektrode einen Hohlraum beinhaltet; wobei der Hohlraum

a) zu dem Umgebungsvolumen hin offen ist, und
b) einen Wirkstoff beinhaltet.

Ausführungsform 19:

Die Vorrichtung nach einer der vorhergehenden Ausführungsformen, wobei der Hohlkörper ein Therapiegerät ist.

Ausführungsform 20:

Die Vorrichtung nach einer der vorhergehenden Ausführungsformen, wobei das erste Bauteil

a) ein Gehäuse ist; und
b) einen Anschlussflansch, beinhaltend eine Flanschöffnung, beinhaltet;

wobei die Flanschöffnung das zweite Bauteil beinhaltet; wobei das zweite Bauteil ein Rahmen, beinhaltend eine Rahmenöffnung, ist; wobei die Elektrode die Rahmenöffnung durchstößt.

Ausführungsform 21:

Die Vorrichtung nach einer der Ausführungsformen 1 bis 19, wobei das erste Bauteil

a) ein Gehäuse ist; und

b) eine Gehäuseöffnung beinhaltet;

wobei die Gehäuseöffnung das zweite Bauteil beinhaltet; wobei das zweite Bauteil ein Rahmen, beinhaltend eine Rahmenöffnung, ist; wobei die Elektrode die Rahmenöffnung durchstößt; wobei der Rahmen einen Metallgehalt beinhaltet; wobei der Metallgehalt des Rahmens radial nach außen zunimmt.

Ausführungsform 22:

Die Vorrichtung nach einer der Ausführungsformen 1 bis 19, wobei das zweite Bauteil ein Gehäuse ist.

Ausführungsform 23:

Verfahren zu einer Herstellung einer Vorrichtung, beinhaltend als Verfahrensschritte

a) Bereitstellen eines Gehäuses, beinhaltend eine Gehäuseöffnung;
b) Einbringen eines elektronischen Bauteils in das Gehäuse durch die Gehäuseöffnung;
c) Verschließen der Gehäuseöffnung mit einem Verschluss;
d) Verbinden des Gehäuses mit einer Elektrode;

wobei die Elektrode ein Cermet beinhaltet, wobei die Elektrode das elektronische Bauteil elektrisch leitend mit einer Umgebung des Gehäuses verbindet, wobei die Elektrode eine Kontaktoberfläche beinhaltet, wobei die Kontaktoberfläche ausgebildet ist zu einem Kontaktieren eines eukaryotisch Gewebes, wobei die Kontaktoberfläche einen maximalen Abstand zu dem elektronischen Bauteil von weniger als 80 mm hat.

Ausführungsform 24:

Das Verfahren nach Ausführungsform 23, wobei das Gehäuse elektrisch leitfähig ist; wobei das Verbinden des Gehäuses mit der Elektrode als Unterschritte beinhaltet

a) Verbinden des Gehäuses mit einem Anschlussflansch, beinhaltend eine Flanschöffnung;
b) Einbringen eines Rahmens, beinhaltend eine Keramik und eine Rahmenöffnung, in die Flanschöffnung;

wobei die Elektrode die Rahmenöffnung durchstößt.

Ausführungsform 25:

Das Verfahren nach Ausführungsform 23, wobei das Gehäuse elektrisch leitfähig ist; wobei das Verbinden des Gehäuses mit der Elektrode als Unterschritte beinhaltet ein Verbinden des Gehäuses mit einem Rahmen, beinhaltend eine Rahmenöffnung, eine Keramik und einen Metallgehalt; wobei die Elektrode die Rahmenöffnung durchstößt, wobei der Metallgehalt des Rahmens radial nach außen zunimmt.

Ausführungsform 26:

Das Verfahren nach Ausführungsform 23, wobei das Gehäuse eine Keramik beinhaltet.

Ausführungsform 27:

Das Verfahren nach einer der Ausführungsformen 23 bis 26, wobei der Verschluss elektrisch leitfähig ist.

Ausführungsform 28:

Vorrichtung erhältlich durch das Verfahren nach einer der Ausführungsformen 23 bis 27.

Ausführungsform 29:

Verwendung einer Elektrode zu einem Herstellen eines kabellosen Herzschrittmachers, wobei die Elektrode ein Cermet beinhaltet.

Messmethoden

Die folgenden Messmethoden wurden im Rahmen der Erfindung benutzt. Sofern nichts anderes angegeben ist wurden die Messungen bei einer Umgebungstemperatur von 25°C, einem Umgebungsluftdruck von 100 kPa (0,986 atm) und einer relativen Luftfeuchtigkeit von 50 % durchgeführt.

innere Oberfläche

Zunächst wird wie unten beschrieben die äußere Oberfläche der Fläche bestimmt. Anschließend wird die Probe in ein Rasterelektronenmikroskop Ultra 55 der Carl Zeiss AG (73447 Oberkochen, Deutschland) zur Vermessung der Fläche positioniert. Zur Bilderzeugung wird die Software SmartSEM (ebenfalls Carl Zeiss AG) genutzt. Über diese Software wird ein Vergrößerungsfaktor 500 sowie eine Anregungsspannung von 20 kV gewählt. Es wird eine Aufnahme, welche vollständig durch die zu vermessende Fläche ausgefüllt ist, erstellt. Das erzeugte Bild wird in MS Paint (Microsoft Deutschland GmbH) geöffnet. Es wird ein Quadrat der Kantenlänge $1\,\mu$m mit Hilfe der Skala der Aufnahme gezeichnet. Dieses Quadrat wird 9 ma1 kopiert, so dass 10 identische Quadrate der Kantenlänge $1\,\mu$m erhalten werden. Die 10 Quadrate werden in 2 Reihen zu je 5 Quadraten untereinander auf der Aufnahme angeordnet von der oberen linken Ecke der Aufnahme angefangen. In jedem Quadrat wird die Anzahl der schwarzen Flächen gezählt. Diese Anzahl wird über die 10 Quadrate gemittelt. Es wird die Porenanzahl pro $1\,\mu m^2$ erhalten. Die Porenanzahl pro $1\,\mu m^2$ wird mit der äußeren Oberfläche in $\mu m^2$ multipliziert und die Anzahl Z der Poren auf der gesamten zu vermessenden Fläche erhalten. Ferner werden 30 beliebige Poren auf der Aufnahme ausgewählt und deren längste Ausdehnung entlang einer Geraden in der Ebene der Aufnahme mit dem Längenvermessungstool der Software SmartSEM bestimmt. Der durchschnittliche Porendurchmesser d wird als Mittelwert über die an den 30 Poren gemessenen Ausdehnungen bestimmt. Der mittlere Porenradius ist demnach r = d / 2. Nun werden die Poren als im Mittel halbkugelförmig angenommen und die innere Oberfläche wird berechnet zu:

$$\text{innere Oberfläche} = \text{äußere Oberfläche} - (Z \cdot \pi r^2) + (Z \cdot 2\pi r^2) = \text{äußere Oberfläche} + Z \cdot \pi r^2.$$

äußere Oberfläche

Die äußere Oberfläche einer Fläche wird bestimmt durch eine geometrische Berechnung der des Flächeninhalts der Fläche.

Rauheit

Die Rauheit wird ermittelt gemäß der Norm EN ISO 4288 : 1997 als die darin beschriebene mittlere Rauheit $R_a$.

elektrische Leitfähigkeit

Die elektrische Leitfähigkeit wird mit einem handelsüblichen Leitfähigkeitsmessgerät (GLF 100 Universal-Leitfähigkeitsmessgerät von GHM Messtechnik GmbH Standort Greisinger, Regenstauf, Deutschland) gemessen.

offene Porendichte

Von der zu vermessenden Fläche wird zunächst eine Rasterelektronenmikroskopaufnahme mit einem Ultra 55 der Carl Zeiss AG (73447 Oberkochen, Deutschland) erstellt. Zur Bilderzeugung wird die Software SmartSEM (ebenfalls Carl Zeiss AG) genutzt. Über diese Software wird ein Vergrößerungsfaktor 500 sowie eine Anregungsspannung von 20 kV gewählt. Das erzeugte Bild wird in MS Paint (Microsoft Deutschland GmbH) geöffnet. Es werden drei Geraden über die Aufnahme gelegt. Jede Gerade beginnt an einer Bildseite und endet an der gegenüberliegenden Bildseite. Für jede der Geraden wird die Anzahl der Poren, welche die Gerade berührt oder schneidet, gezählt. Die Länge jeder Geraden wird bestimmt mit Hilfe der Skala der Aufnahme bestimmt. 1 Inch wird durch die Länge der Geraden geteilt und die gezählte Porenanzahl mit diesem Faktor multipliziert. Somit wird für jede der drei Geraden eine Porendichte in ppi erhalten. Der Mittelwert dieser drei Werte ist die zu bestimmende offene Porendichte in ppi.

Biokompatibilität

Die Biokompatibilität wird gemäß der Norm nach 10993-4:2002 bestimmt.

elektrische Kapazität

Zur Messung der elektrischen Kapazität einer Elektrode wird zunächst ein Becherglas ausreichenden Volumens mit vollentsalztem Wasser mit 0,9 Gew.-% NaCl bezogen auf die Lösung befüllt. Anschließend wird ein gebogenes Edelstahlblech als Gegenelektrode so über den Becherglasrand gehängt, dass die Gegenelektrode teilweise in das Wasser eintaucht. Dann wird das Becherglas in ein beheiztes Wasserbad gestellt und auf 37°C temperiert. Dann wird die zu vermessende Elektrode mit der Seite, an welcher sich die Kontaktoberfläche befindet, in die Lösung getaucht. Die gegenüberliegende Seite der Elektrode wird nicht in die Lösung getaucht. Anschließend wird der Versuchsaufbau für 24 h so belassen. Nach den 24 h wird eine handelsübliche Referenzelektrode, erhältlich bei Radiometer Analytical (REF621), in die Lösung getaucht. Es wird ein Potentiostat (Gambry Instrumentation Reference 600) bereitgestellt und die Klammern des Potentiostats folgendermaßen angeschlossen. Die grüne und blaue Klammer werden kurzgeschlossen und an der aus der Lösung ragenden Seite der zu vermessenden Elektrode angeschlossen. Die weiße Klammer wird an die Referenzelektrode angeschlossen. Die orangene und die rote Klammer werden kurzgeschlossen und mit der Gegenelektrode verbunden. Die schwarze Klammer dient zur Erdung. Zu Datenaufnahme wird die Software Gambry Instrumentation Framework verwendet. Hierin gelangt man über die Reiter Analysis, Electromechnanical Impedance und Potentostatic EIS zur gewünschten Anwendung. Folgenden Parameter sind einzugeben: Initial Frequency: 100000 Hz; Final Frequency: 0,1 Hz; 10 Points; 10 mV. Die Messung wird gestartet. Es wird von der Software ein Bode-Diagramm erzeugt, aus dem die Impedanz bei einer Frequenz von 0,1 Hz abgelesen werden kann. Die Kapazität berechnet sich zu Kapazität = 1 / (Impedanz·2·$\pi$·0,1).

Die Erfindung wird im Folgenden durch Beispiele und Zeichnungen genauer dargestellt, wobei die Beispiele und Zeichnungen keine Einschränkung der Erfindung bedeuten. Es zeigen:

Figur 1       einen schematischen Querschnitt einer Ausführungsform einer erfindungsgemäßen Vorrichtung in Seitenansicht;

Figur 2a      einen schematischen Querschnitt einer weiteren Ausführungsform einer erfindungsgemäßen Vorrichtung in Seitenansicht;

Figur 2b      eine schematische Ansicht der Elektrode und des zweiten Bauteils der Ausführungsform in Figur 2a von dem Umgebungsvolumen aus;

Figur 3       einen schematischen Querschnitt einer weiteren Ausführungsform einer erfindungsgemäßen Vorrichtung in Seitenansicht;

Figur 4       einen schematischen Querschnitt einer weiteren Ausführungsform einer erfindungsgemäßen Vorrichtung in Seitenansicht;

Figur 5       einen schematischen Querschnitt einer weiteren Ausführungsform einer erfindungsgemäßen Vorrichtung in Seitenansicht;

Figur 6a      einen schematischen Querschnitt einer weiteren Ausführungsform einer erfindungsgemäßen Vorrichtung in Seitenansicht;

Figur 6b      eine schematische Ansicht der Elektrode der Ausführungsform in Figur 6a von dem Umgebungsvolumen aus;

Figur 6c      eine schematische Ansicht der Elektrode der Ausführungsform in Figur 6a von dem Innenvolumen aus;

Figur 7a      einen schematischen Querschnitt einer weiteren Ausführungsform einer erfindungsgemäßen Vorrichtung in Seitenansicht;

Figur 7b      eine schematische Ansicht der Elektrode der Ausführungsform in Figur 7a von dem Umgebungsvolumen aus;

Figur 8       einen schematischen Querschnitt eines nicht erfindungsgemäßen kabellosen Herzschrittmachers in Seitenansicht; und

Figur 9       einen schematischen Querschnitt einer weiteren Ausführungsform einer erfindungsgemäßen Vorrichtung in Seitenansicht.

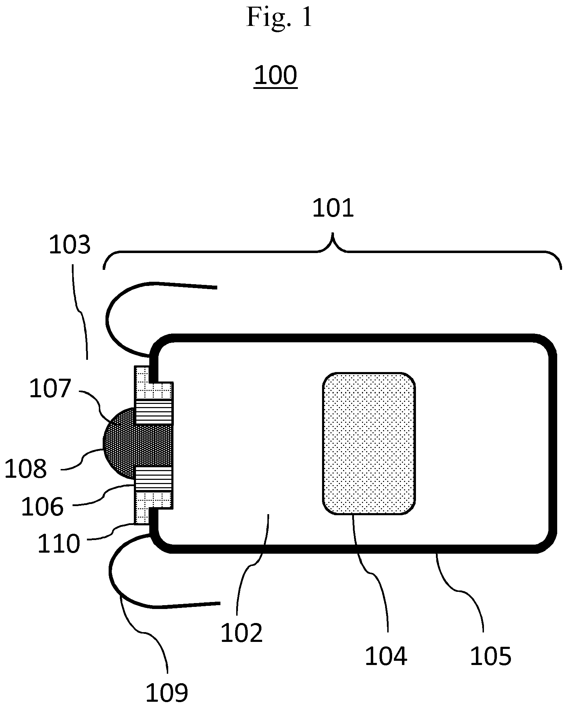

Figur 1 zeigt einen schematischen Querschnitt einer Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104. Der Hohlkörper 101 beinhaltet ein erstes Bauteil 105, hier ein Gehäuse 105; ein zweites Bauteil 106, hier ein Keramikring 106; und eine Elektrode 107. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist ein Anschlussflansch 110 eingeschweißt. Der Anschlussflansch 110 besteht aus einer biokompatiblen Titan-Legierung für medizinische Zwecke (erhältlich bei Hempel Special Metals AG). Eine Flanschöffnung des Anschlussflansches 110 beinhaltet den Keramikring 106. Der Keramikring 106 ist mit einem Gold-Lot in die Flanschöffnung eingelötet. Der Keramikring 106 isoliert das Gehäuse 105 und den Anschlussflansch 110 elektrisch von der Elektrode 107. Der Keramikring besteht aus $Al_2O_3$. Die Elektrode 107 durchstößt eine Ringöffnung des Keramikrings 106. Die Elektrode 107 beinhaltet ein Cermet. Das Cermet besteht aus 45 Gew.-%

Platinpulver der Firma Heraeus Precious Metals GmbH & Co.KG mit einer Korngröße $D_{50}$ = 50 $\mu$m, und 45 Gew.-% Aluminiumoxid ($Al_2O_3$) der Firma CeramTech GmbH mit einer Korngröße von $D_{90}$ = 2 $\mu$m, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode 107 ist einstückig ausgebildet. Die Elektrode 107 ist ein starrer Körper. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und der Keramikring 106 wurden gemeinsam einstückig in einem Sinterprozess hergestellt. Der Hohlkörper 101 beinhaltet weiter ein Fixierelement 109, hier einen Haken 109, an seiner Außenseite. Der Haken 109 ist ausgebildet zu einem Fixieren einer Position des Hohlkörpers 101 relativ zu dem menschlichen Herzmuskelgewebe. Der Hohlkörper 101 trennt das Innenvolumen 102 hermetisch dicht von dem Umgebungsvolumen 103. Der Hohlkörper 101 ist ein kabelloser Herzschrittmacher 101.

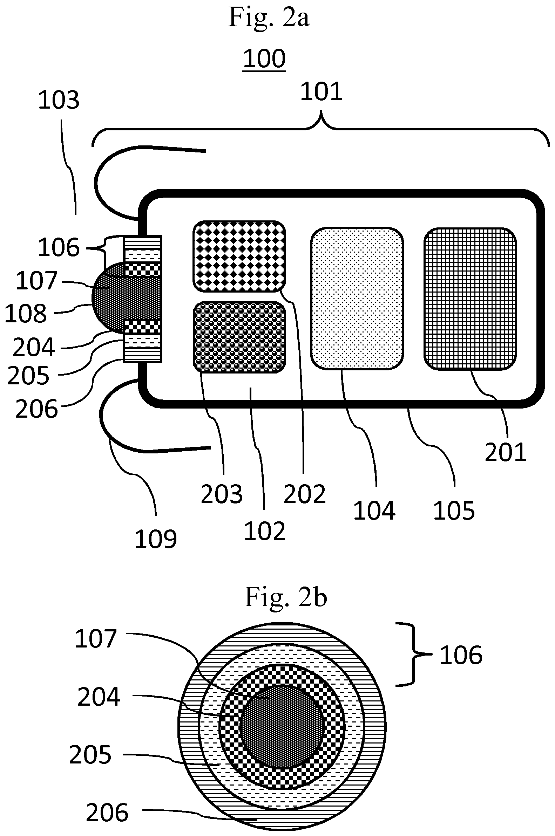

Figur 2a zeigt einen schematischen Querschnitt einer weiteren Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104; eine Energieversorgung 201, hier eine Lithium-Batterie 201; eine Steuereinheit 202, hier eine programmierbare Steuereinheit 202; und eine Messeinheit 203. Der Hohlkörper 101 beinhaltet ein erstes Bauteil 105, hier ein Gehäuse 105; ein zweites Bauteil 106, hier ein Hohlzylinder 106; und eine Elektrode 107. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist der Hohlzylinder 106 eingeschweißt. Der Hohlzylinder 106 besteht aus einer Keramik, beinhaltend einen Metallgehalt, wobei der Metallgehalt des Hohlzylinders 106 radial nach außen zunimmt. Der Metallgehalt des Hohlzylinders 106 nimmt radial nach außen in drei Stufen zu. Der Hohlzylinder 106 beinhaltet drei konzentrische Teilhohlzylinder 204, 205 und 206. Der innere Teilhohlzylinder 204 beinhaltet einen Metallgehalt von 0 Gew.-% bezogen auf das Gesamtgewicht des inneren Teilhohlzylinders 204. Der mittlere Teilhohlzylinder 205 beinhaltet einen Metallgehalt von 40 Gew.-% bezogen auf das Gesamtgewicht des mittleren Teilhohlzylinders 205. Der äußere Teilhohlzylinder 206 beinhaltet einen Metallgehalt von 80 Gew.-% bezogen auf das Gesamtgewicht des äußeren Teilhohlzylinders 206. Das Metall, auf das sich der Metallgehalt bezieht ist dabei Platin. Die Elektrode 107 durchstößt eine Hohlzylinderöffnung des Hohlzylinders 106. Die Elektrode 107 beinhaltet ein Cermet. Das Cermet besteht aus 45 Gew.% Platinpulver der Firma Heraeus Precious Metals GmbH & Co.KG mit einer Korngröße $D_{50}$ = 50 $\mu$m, und 45 Gew.-% Aluminiumoxid ($Al_2O_3$) der Firma CeramTech GmbH mit einer Korngröße von $D_{90}$ = 2 $\mu$m, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode 107 ist einstückig ausgebildet. Die Elektrode 107 ist ein starrer Körper. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und der Hohlzylinder 106 wurden gemeinsam einstückig in einem Sinterprozess hergestellt. Der Hohlkörper 101 beinhaltet weiter ein Fixierelement 109, hier ein Haken 109, an seiner Außenseite. Der Haken 109 ist ausgebildet zu einem Fixieren einer Position des Hohlkörpers 101 relativ zu dem menschlichen Herzmuskelgewebe. Der Hohlkörper 101 trennt das Innenvolumen 102 hermetisch dicht von dem Umgebungsvolumen 103. Der Hohlkörper 101 ist ein kabelloser Herzschrittmacher 101.

Figur 2b zeigt eine schematische Ansicht der Elektrode 107 und des zweiten Bauteils 106 der Ausführungsform in Figur 2a von dem Umgebungsvolumen 103 aus. Das zweite Bauteil 106 ist hier ein Hohlzylinder 106. Der Hohlzylinder 106 setzt sich aus drei konzentrischen Teilhohlzylindern 204, 205 und 206 zusammen. Der innere Teilhohlzylinder 204 beinhaltet einen Metallgehalt von 0 Gew.-% bezogen auf das Gesamtgewicht des inneren Teilhohlzylinders 204. Der mittlere Teilhohlzylinder 205 beinhaltet einen Metallgehalt von 40 Gew.-% bezogen auf das Gesamtgewicht des mittleren Teilhohlzylinders 205. Der äußere Teilhohlzylinder 206 beinhaltet einen Metallgehalt von 80 Gew.-% bezogen auf das Gesamtgewicht des äußeren Teilhohlzylinders 206.

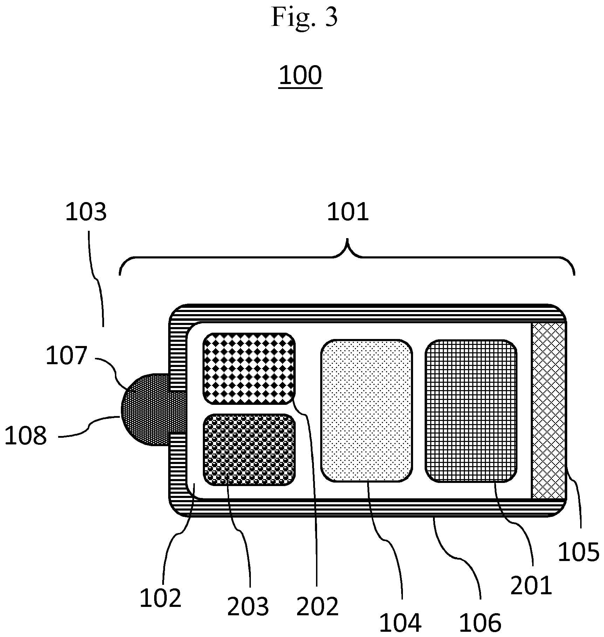

Figur 3 zeigt einen schematischen Querschnitt einer weiteren Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104; eine Energieversorgung 201, hier eine Lithium-Batterie 201; eine Steuereinheit 202, hier eine programmierbare Steuereinheit 202; und eine Messeinheit 203. Der Hohlkörper 101 beinhaltet ein erstes Bauteil 105, hier ein Verschluss 105; ein zweites Bauteil 106, hier ein Gehäuse 106; und eine Elektrode 107. Der Verschluss 105 ist elektrisch leitend. Der Verschluss 105 ist in das Gehäuse 106 eingelötet mit einem Gold-Lot. Der Verschluss 105 besteht aus einer biokompatiblen Titan-Legierung für medizinische Zwecke (erhältlich bei Hempel Special Metals AG). Das Gehäuse 106 ist elektrisch isolierend. Das Gehäuse besteht aus einer Keramik. Die Keramik beinhaltet $Al_2O_3$. Die Elektrode 107 durchstößt eine Gehäuseöffnung des Gehäuses 106. Die Elektrode 107 beinhaltet ein Cermet. Das Cermet besteht aus 45 Gew.-% Platinpulver der Firma Heraeus Precious Metals GmbH & Co.KG mit einer Korngröße $D_{50} = 50$ $\mu$m, und 45 Gew.-% Aluminiumoxid ($Al_2O_3$) der Firma CeramTech GmbH mit einer Korngröße von $D_{90} = 2$ $\mu$m, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode 107 ist einstückig ausgebildet. Die Elektrode 107 ist ein starrer Körper. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und das Gehäuse 106 wurden gemeinsam einstückig in einem Sinterprozess hergestellt. Der Hohlkörper 101 trennt das Innenvolumen 102 hermetisch dicht von dem Umgebungsvolumen 103. Der Hohlkörper 101 ist ein kabelloser Herzschrittmacher 101.

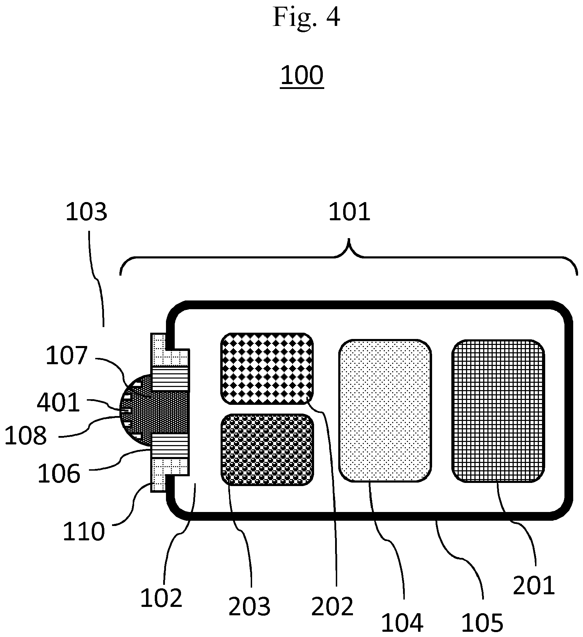

Figur 4 zeigt einen schematischen Querschnitt einer weiteren Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104; eine Energieversorgung 201, hier eine Lithium-Batterie 201; eine Steuereinheit 202, hier eine programmierbare Steuereinheit 202; und eine Messeinheit 203. Der Hohlkörper 101 beinhaltet ein erstes Bauteil 105, hier ein Gehäuse 105; ein zweites Bauteil 106, hier ein Keramikring 106; und eine Elektrode 107. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist ein Anschlussflansch 110 eingeschweißt. Der Anschlussflansch 110 besteht aus einer biokompatiblen Titan-Legierung für medizinische Zwecke (erhältlich bei Hempel Special Metals AG). Eine Flanschöffnung des Anschlussflansches 110 beinhaltet den Keramikring 106. Der Keramikring 106 ist mit einem Gold-Lot in die Flanschöffnung eingelötet. Der Keramikring 106 isoliert das Gehäuse 105 und den Anschlussflansch 110 elektrisch von der Elektrode 107. Der Keramikring 106 besteht aus $Al_2O_3$. Die Elektrode 107 durchstößt eine Ringöffnung des Keramikrings 106. Die Elektrode 107 beinhaltet ein Cermet. Das Cermet besteht aus 45 Gew.-% Platinpulver der Firma Heraeus Precious Metals GmbH & Co.KG mit einer Korngröße $D_{50} = 50$ $\mu$m, und 45 Gew.-% Aluminiumoxid ($Al_2O_3$) der Firma CeramTech GmbH mit einer Korngröße von $D_{90} = 2$ $\mu$m, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode 107 ist einstückig ausgebildet. Die Elektrode 107 ist ein starrer Körper. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und der Keramikring 106 wurden gemeinsam einstückig in einem Sinterprozess hergestellt. Die Elektrode 107 beinhaltet eine Pluralität von Hohlräumen 401, die zu dem Umgebungsvolumen 103 hin offen sind. Die Hohlräume 401 sind Bohrungen 401. Die Bohrungen 401 sind auf der Kontaktoberfläche 108 verteilt. Jede der Bohrungen 401 beinhaltet ein Medikament, beinhaltend einen steroidalen Entzündungshemmer. Der Hohlkörper 101 trennt das Innenvolumen 102 hermetisch dicht von dem Umgebungsvolumen 103. Der Hohlkörper 101 ist ein kabelloser Herzschrittmacher 101.

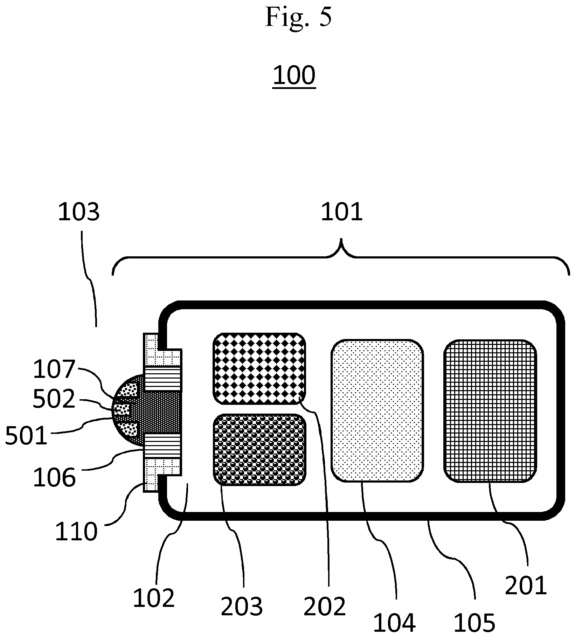

Figur 5 zeigt einen schematischen Querschnitt einer weiteren Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104; eine Energieversorgung 201, hier eine Lithium-Batterie 201; eine Steuereinheit 202, hier eine programmierbare Steuereinheit 202; und eine Messeinheit 203. Der Hohlkörper 101 beinhaltet ein erstes Bauteil 105, hier ein Gehäuse 105; ein zweites Bauteil 106, hier ein Keramikring 106; und eine Elektrode 107. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist ein Anschlussflansch 110 eingeschweißt. Der Anschlussflansch 110 besteht aus einer biokompatiblen Titan-Legierung für medizinische Zwecke (erhältlich bei Hempel Special Metals AG). Eine Flanschöffnung des Anschlussflansches 110 beinhaltet den Keramikring 106. Der Keramikring 106 ist mit einem Gold-Lot in die Flanschöffnung eingelötet. Der Keramikring 106 isoliert das Gehäuse 105 und den Anschlussflansch 110 elektrisch von der Elektrode 107. Der Keramikring 106 besteht aus $Al_2O_3$. Die Elektrode 107 durchstößt eine Ringöffnung des Keramikrings 106. Die Elektrode 107 beinhaltet ein Cermet. Das Cermet besteht aus 45 Gew.-% Platinpulver der Firma Heraeus Precious Metals GmbH & Co.KG mit einer Korngröße $D_{50} = 50$ $\mu$m, und 45 Gew.-% Aluminiumoxid ($Al_2O_3$) der Firma CeramTech GmbH mit

einer Korngröße von $D_{90}$ = 2 μm, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode 107 ist einstückig ausgebildet. Die Elektrode 107 ist ein starrer Körper. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und der Keramikring 106 wurden gemeinsam einstückig in einem Sinterprozess hergestellt. Die Kontaktoberfläche 108 beinhaltet elektrisch leitende Teiloberflächen 501 und elektrisch isolierende Teiloberflächen 502. Die innere Oberfläche aller elektrisch leitenden Teiloberflächen 501 beträgt 25 mm². Die äußere Oberfläche aller elektrisch leitenden Teiloberflächen 501 beträgt 5 mm². Die äußere Oberfläche aller elektrisch isolierenden Teiloberflächen 502 beträgt 20 mm². Die gesamte äußere Oberfläche der Kontaktoberfläche 108 ist so groß, dass die Elektrode 107 das Herzmuskelgewebe nicht perforiert. Der Hohlkörper 101 trennt das Innenvolumen 102 hermetisch dicht von dem Umgebungsvolumen 103. Der Hohlkörper 101 ist ein kabelloser Herzschrittmacher 101.

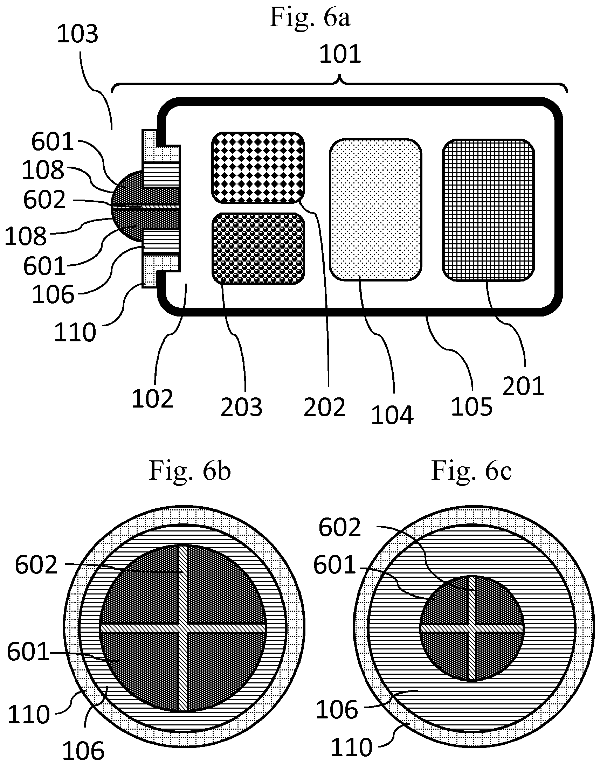

Figur 6a zeigt einen schematischen Querschnitt einer weiteren Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104; eine Energieversorgung 201, hier eine Lithium-Batterie 201; eine Steuereinheit 202, hier eine programmierbare Steuereinheit 202; und eine Messeinheit 203. Der Hohlkörper 101 beinhaltet ein erstes Bauteil 105, hier ein Gehäuse 105; ein zweites Bauteil 106, hier ein Keramikring 106; und eine Elektrode 107. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist ein Anschlussflansch 110 eingeschweißt. Der Anschlussflansch 110 besteht aus einer biokompatiblen Titan-Legierung für medizinische Zwecke (erhältlich bei Hempel Special Metals AG). Eine Flanschöffnung des Anschlussflansches 110 beinhaltet den Keramikring 106. Der Keramikring 106 ist mit einem Gold-Lot in die Flanschöffnung eingelötet. Der Keramikring 106 isoliert das Gehäuse 105 und den Anschlussflansch 110 elektrisch von der Elektrode 107. Der Keramikring 106 besteht aus $Al_2O_3$. Die Elektrode 107 durchstößt eine Ringöffnung des Keramikrings 106. Die Elektrode 107 beinhaltet ein Cermet. Das Cermet besteht aus 45 Gew.-% Platinpulver der Firma Heraeus Precious Metals GmbH & Co.KG mit einer Korngröße $D_{50}$ = 50 μm, und 45 Gew.-% Aluminiumoxid ($Al_2O_3$) der Firma CeramTech GmbH mit einer Korngröße von $D_{90}$ = 2 μm, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und der Keramikring 106 wurden gemeinsam einstückig in einem Sinterprozess hergestellt. Die Elektrode 107 beinhaltet vier Teilelektroden 601. Jede der Teilelektroden 601 verbindet das Innenvolumen 102 elektrisch leitend mit dem Umgebungsvolumen 103. Jede Teilelektrode 601 ist von den jeweils anderen Teilelektroden 601 elektrisch isoliert durch eine Keramik 602. Jede der Teilelektroden 601 ist einstückig ausgebildet. Jede der Teilelektroden 601 ist ein starrer Körper. Der Hohlkörper 101 trennt das Innenvolumen 102 hermetisch dicht von dem Umgebungsvolumen 103. Der Hohlkörper 101 ist ein kabelloser Herzschrittmacher 101.

Figur 6b zeigt eine schematische Ansicht der Elektrode 107 der Ausführungsform in Figur 6a von dem Umgebungsvolumen 103 aus. Zu sehen ist ferner der Keramikring 106 und der Anschlussflansch 110 sowie die Keramik 602, welche die Teilelektroden 601 voneinander elektrisch isoliert.

Figur 6c zeigt eine schematische Ansicht der Elektrode 107 der Ausführungsform in Figur 6a von dem Innenvolumen 102 aus. Zu sehen ist ferner der Keramikring 106 und der Anschlussflansch 110 sowie die Keramik 602, welche die Teilelektroden 601 voneinander elektrisch isoliert.

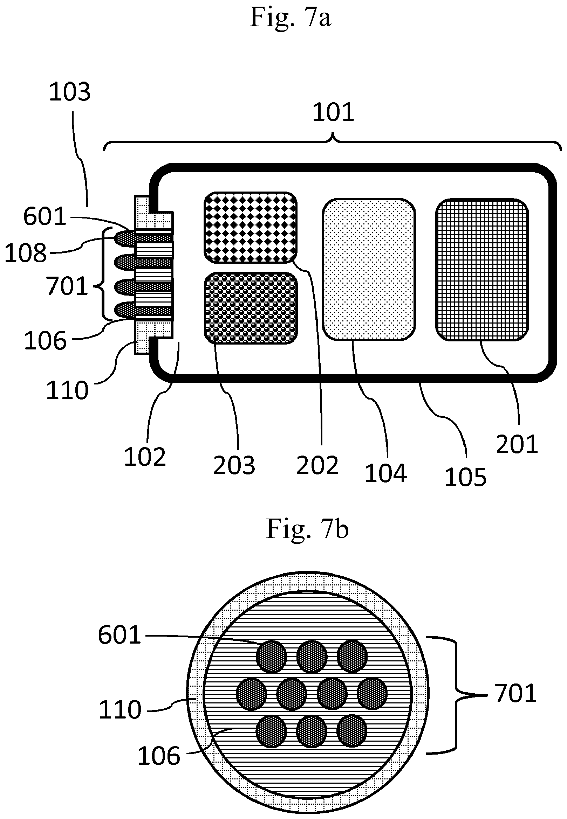

Figur 7a zeigt einen schematischen Querschnitt einer weiteren Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein elektrischer Impulsgenerator 104; eine Energieversorgung 201, hier eine Lithium-Batterie 201; eine Steuereinheit 202, hier eine programmierbare Steuereinheit 202; und eine Messeinheit 203. Der Hohlkörper 101 beinhaltet ein erstes Bauteil 105, hier ein Gehäuse 105; ein zweites Bauteil 106, hier eine Lochplatte 106; und eine Elektrode 107, wobei die Elektrode 107 aus einer Pluralität von Teilelektroden 601 besteht. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist ein Anschlussflansch 110 eingeschweißt. Der Anschlussflansch 110 besteht aus einer biokompatiblen Titan-Legierung für medizinische Zwecke (erhältlich bei Hempel Special Metals AG). Eine Flanschöffnung des Anschlussflansches 110 beinhaltet die Lochplatte 106. Die Lochplatte 106 ist mit einem Gold-Lot in die Flanschöffnung eingelötet. Die Lochplatte 106 isoliert das Gehäuse 105 und den Anschlussflansch 110 elektrisch von der Elektrode 107. Die Lochplatte 106 besteht aus $Al_2O_3$. Je eine Teilelektrode 601 durchstößt ein Loch der Lochplatte 106. Die Teilelektroden 601 und damit die Elektrode 107 beinhaltet ein Cermet. Das Cermet besteht aus 45 Gew.-% Platinpulver der Firma Heraeus Precious Metals GmbH & Co.KG mit einer Korngröße $D_{50}$ = 50 $\mu$m, und 45 Gew.-% Aluminiumoxid ($Al_2O_3$) der Firma CeramTech GmbH mit einer Korngröße von $D_{90}$ = 2 $\mu$m, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Jede der Teilelektroden 601 verbindet das Innenvolumen 102 elektrisch leitend mit dem Umgebungsvolumen 103. Jede Teilelektrode 601 ist von den jeweils anderen Teilelektroden 601 elektrisch isoliert durch die Lochplatte 106. Jede der Teilelektroden 601 ist einstückig ausgebildet. Jede der Teilelektroden 601 ist ein starrer Körper. Die Teilelektroden 601 bilden ein Array von Teilelektroden 701. Die Elektrode 107 beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Die Elektrode 107 und die Lochplatte 106 wurden gemeinsam einstückig in einem Sinterprozess hergestellt. Der Hohlkörper 101 trennt das Innenvolumen 102 hermetisch dicht von dem Umgebungsvolumen 103. Der Hohlkörper 101 ist ein kabelloser Herzschrittmacher 101.

Figur 7b zeigt eine schematische Ansicht der Elektrode 107 der Ausführungsform in Figur 7a von dem Umgebungsvolumen 103 aus. Zu sehen ist ferner die Lochplatte 106, welche die Teilelektroden 601 voneinander elektrisch isoliert, und der Anschlussflansch 110.

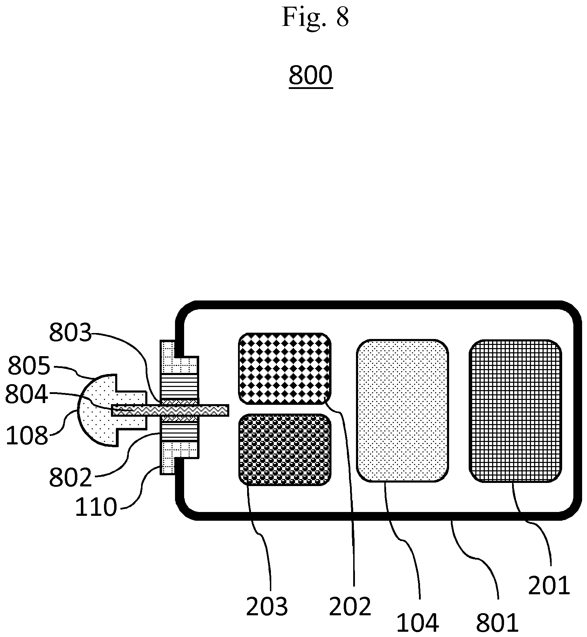

Figur 8 zeigt einen schematischen Querschnitt eines nicht erfindungsgemäßen kabellosen Herzschrittmachers 800 in Seitenansicht. Der kabellose Herzschrittmacher 800 beinhaltet ein Gehäuse 801, welches eine Energieversorgung 201, hier eine Batterie 201; ein elektronisches Bauteil 104, hier ein elektrischer Impulsgenerator 104; eine Steuereinheit 202; und eine Messeinheit 203 umschließt. In eine Gehäuseöffnung des Gehäuses 801 ist ein Anschlussflansch 110 eingeschweißt. In eine Flanschöffnung des Anschlussflansches 110 ist ein Keramikring 802 mit einem Gold-Lot eingelötet. In eine Ringöffnung des Keramikrings 802 ist ein Durchführungsdraht 804 mit einem Gold-Lot 803 eingelötet. An ein außerhalb des Gehäuses 801 liegendes Ende des Durchführungsdrahts 804 ist ein Kontaktkörper 805 angeschweißt. Der Kontaktkörper 805 beinhaltet eine Kontaktoberfläche 108. Die Kontaktoberfläche 108 ist ausgebildet zu einem Kontaktieren eines eukaryotischen Gewebes. Die Kontaktoberfläche 108 ist ausgebildet zu einem Kontaktieren eines menschlichen Herzmuskelgewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem elektrischen Impulsgenerator 104 beträgt etwa 50 mm. Der Durchführungsdraht 804 und der Kontaktkörper 805 bilden zusammen eine nicht erfindungsgemäße Elektrode. Die nicht erfindungsgemäße Elektrode besteht aus einer Platin-Iridium-Legierung. Die nicht erfindungsgemäße Elektrode ist als starrer Körper und mehrstückig ausgebildet.

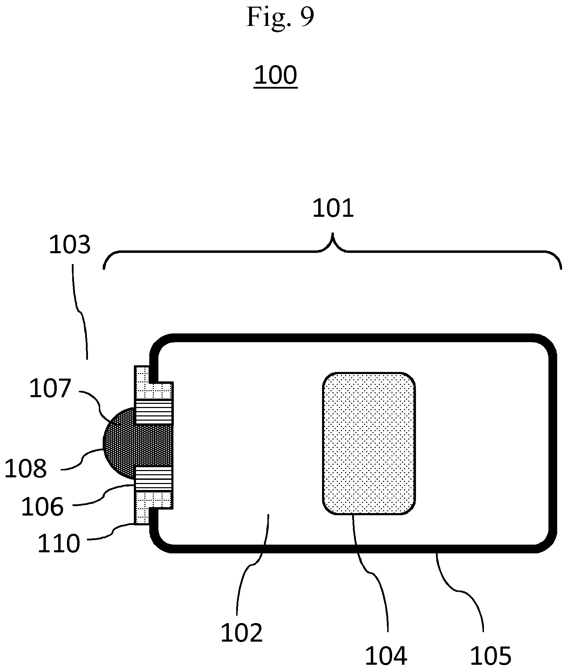

Figur 9 zeigt einen schematischen Querschnitt einer Ausführungsform einer erfindungsgemäßen Vorrichtung 100 in Seitenansicht. Die Vorrichtung 100 beinhaltet einen Hohlkörper 101, ein Innenvolumen 102 und ein Umgebungsvolumen 103. Der Hohlkörper 101 umschließt das Innenvolumen 102. Das Innenvolumen 102 beinhaltet ein elektronisches Bauteil 104, hier ein EKG-Gerät 104. Der Hohlkörper 101 beinhaltet ein erstes Bauteil 105, hier ein Gehäuse 105; ein zweites Bauteil 106, hier ein Keramikring 106; und eine Elektrode 107. Das Gehäuse 105 ist elektrisch leitend. Das Gehäuse besteht aus einer biokompatiblen Titan-Legierung für medizinische Zwecke (erhältlich bei Hempel Special Metals AG). In eine Gehäuseöffnung ist ein Anschlussflansch 110 eingeschweißt. Der Anschlussflansch 110 besteht aus einer biokompatiblen Titan-Legierung für medizinische Zwecke (erhältlich bei Hempel Special Metals AG). Eine Flanschöffnung des Anschlussflansches 110 beinhaltet den Keramikring 106. Der Keramikring 106 ist mit einem Gold-Lot in die Flanschöffnung eingelötet. Der Keramikring 106 isoliert das Gehäuse 105 und den Anschlussflansch 110 elektrisch von der Elektrode 107. Der Keramikring besteht aus $Al_2O_3$. Die Elektrode 107 durchstößt eine Ringöffnung des Keramikrings 106. Die Elektrode 107 beinhaltet ein Cermet. Das Cermet besteht aus 45 Gew.-% Platinpulver der Firma Heraeus Precious Metals GmbH & Co.KG mit einer Korngröße $D_{50}$ = 50 $\mu$m, und 45 Gew.-% Aluminiumoxid ($Al_2O_3$) der Firma CeramTech GmbH mit einer Korngröße von $D_{90}$ = 2 $\mu$m, sowie 10 Gew.-% eines Bindemittels METAWAX P-50 erhältlich bei der Firma Zschimmer & Schwarz GmbH & Co.KG, jeweils bezogen auf das Gesamtgewicht des Cermets. Die Elektrode 107 verbindet das Innenvolumen 102 elektrisch leitend mit dem Umgebungsvolumen 103. Die Elektrode 107 ist einstückig ausgebildet. Die Elektrode 107 ist ein starrer Körper. Die Elektrode beinhaltet eine Kontaktoberfläche 108. Die Kontaktoberfläche 108 ist ausgebildet zu einem Kontaktieren eines eukaryotischen Gewebes. Die Kontaktoberfläche 108 ist dem Umgebungsvolumen zugewandt und zu dem Umgebungsvolumen hin ausgewölbt. Ein maximaler Abstand der Kontaktoberfläche 108 von dem EKG-Gerät 104 beträgt etwa 50 mm. Die Elektrode 107 und der Keramikring 106 wurden gemeinsam einstückig in einem Sinterprozess hergestellt. Der Hohlkörper 101 trennt das Innenvolumen 102 hermetisch dicht von dem Umgebungsvolumen 103. Der Hohlkörper 101 ist ein implantierbarer Biomonitor 101.

Liste der Bezugszeichen

| | |
|---|---|
| 100 | erfindungsgemäße Vorrichtung |
| 101 | Hohlkörper |
| 102 | Innenvolumen |
| 103 | Umgebungsvolumen |
| 104 | elektronisches Bauteil |
| 105 | erstes Bauteil |
| 106 | zweites Bauteil |
| 107 | Elektrode |
| 108 | Kontaktoberfläche |
| 109 | Fixierelement |
| 110 | Anschlussflansch |
| 201 | Energieversorgung |
| 202 | Steuereinheit |
| 203 | Messeinheit |
| 204 | innerer Teilhohlzylinder |
| 205 | mittlerer Teilhohlzylinder |
| 206 | äußerer Teilhohlzylinder |
| 401 | Hohlraum |
| 501 | elektrisch leitende Teiloberfläche |
| 502 | elektrisch isolierende Teiloberfläche |
| 601 | Teilelektrode |
| 602 | Keramik |
| 701 | Array von Teilelektroden |
| 800 | nichterfindungsgemäßer kabelloser Herzschrittmacher |
| 801 | Gehäuse |
| 802 | Keramikring |
| 803 | Gold-Lot |
| 804 | Durchführungsdraht |
| 805 | Kontaktkörper |

**Patentansprüche**

1. Vorrichtung (100) beinhaltend einen Hohlkörper (101), ein Innenvolumen (102) und ein Umgebungsvolumen (103); wobei

   a) das Innenvolumen (102) ein elektronisches Bauteil (104) beinhaltet,
   b) wobei der Hohlkörper (101)

      i) das Innenvolumen (102) umschließt,
      ii) ein erstes Bauteil (105), ein zweites Bauteil (106) und eine Elektrode (107) beinhaltet,

   c) das erste Bauteil (105) elektrisch leitfähig ist;
   d) das zweite Bauteil (106) die Elektrode (107) elektrisch von dem ersten Bauteil (105) isoliert;
   e) die Elektrode (107)

      i) ein Cermet beinhaltet,
      ii) das Innenvolumen (102) elektrisch leitend mit dem Umgebungsvolumen (103) verbindet,
      iii) eine Kontaktoberfläche (108) beinhaltet,

   f) die Kontaktoberfläche (108)

i) ausgebildet ist zu einem Kontaktieren eines eukaryotischen Gewebes,
ii) einen maximalen Abstand zu dem elektronischen Bauteil (104) von weniger als 80 mm hat,

wobei die Elektrode (107) einstückig ausgebildet ist;
wobei die Vorrichtung (100) das elektronische Bauteil (104), das erste Bauteil (105), das zweite Bauteil (106) und die Elektrode (107) beinhaltet,
**dadurch gekennzeichnet dass** die Kontaktoberfläche (108) oder eine Teiloberfläche (501, 502) der Kontaktoberfläche (108) oder beide eine offene Porendichte in einem Bereich von 1000 bis 80000 ppi hat.

2. Die Vorrichtung (100) nach Anspruch 1, wobei das elektronische Bauteil (104) ein elektrischer Impulsgenerator ist.

3. Die Vorrichtung (100) nach einem der vorhergehenden Ansprüche, wobei die Elektrode (107) als starrer Körper ausgebildet ist.

4. Die Vorrichtung (100) nach einem der vorhergehenden Ansprüche, wobei das Cermet ein Metall zu mindestens 25 Vol.-% basierend auf dem Volumen der Elektrode (107) beinhaltet.

5. Die Vorrichtung (100) nach einem der vorhergehenden Ansprüche, wobei das zweite Bauteil (106) eine Keramik beinhaltet.

6. Die Vorrichtung (100) nach einem der vorhergehenden Ansprüche, wobei die Kontaktoberfläche (108) eine elektrisch leitende Teiloberfläche beinhaltet,
wobei die elektrisch leitende Teiloberfläche eine äußere Oberfläche kleiner als 25 mm$^2$ hat.

7. Die Vorrichtung (100) nach einem der vorhergehenden Ansprüche, wobei die Kontaktoberfläche (108) eine elektrisch leitende Teiloberfläche (501) beinhaltet,
wobei eine innere Oberfläche der elektrisch leitenden Teiloberfläche (501) mindestens doppelt so groß ist wie die äußere Oberfläche der elektrisch leitenden Teiloberfläche (501).

8. Die Vorrichtung (100) nach einem der vorhergehenden Ansprüche, wobei die Kontaktoberfläche (108) oder eine Teiloberfläche (501, 502) der Kontaktoberfläche (108) oder beide eine mittlere Rauheit in einem Bereich von 0,2 bis 8 µm hat.

9. Die Vorrichtung (100) nach einem der vorhergehenden Ansprüche, wobei der Hohlkörper (101) ein Therapiegerät ist.

10. Verfahren zu einer Herstellung einer Vorrichtung (100), beinhaltend als Verfahrensschritte

a) Bereitstellen eines Gehäuses, beinhaltend eine Gehäuseöffnung;
b) Einbringen eines elektronischen Bauteils (104) in das Gehäuse durch die Gehäuseöffnung;
c) Verschließen der Gehäuseöffnung mit einem Verschluss;
d) Verbinden des Gehäuses mit einer Elektrode (107);
wobei die Elektrode (107) ein Cermet beinhaltet,
wobei die Elektrode (107) das elektronische Bauteil elektrisch leitend mit einer Umgebung des Gehäuses verbindet,
wobei die Elektrode (107) eine Kontaktoberfläche (108) beinhaltet,
wobei die Kontaktoberfläche (108) ausgebildet ist zu einem Kontaktieren eines eukaryotischen Gewebes,
wobei die Kontaktoberfläche (108) einen maximalen Abstand zu dem elektronischen Bauteil (104) von weniger als 80 mm hat,
**dadurch gekennzeichnet dass** das Verfahren einen weiteren Verfahrensschritt beinhaltet, in dem eine offene Porendichte der Kontaktoberfläche (108) oder einer Teiloberfläche der Kontaktoberfläche (108) oder beider durch ein Ätzen der entsprechenden Oberfläche vergrößert wird.

11. Das Verfahren nach Anspruch 10, wobei das Gehäuse elektrisch leitfähig ist;

wobei das Verbinden des Gehäuses mit der Elektrode (107) als Unterschritte beinhaltet

a) Verbinden des Gehäuses mit einem Anschlussflansch (110), beinhaltend eine Flanschöffnung;
b) Einbringen eines Rahmens, beinhaltend eine Keramik und eine Rahmenöffnung, in die Flanschöffnung;

wobei die Elektrode (107) die Rahmenöffnung durchstößt.

12. Das Verfahren nach Anspruch 10, wobei das Gehäuse elektrisch leitfähig ist;

wobei das Verbinden des Gehäuses mit der Elektrode (107) als Unterschritte beinhaltet ein Verbinden des Gehäuses mit einem Rahmen, beinhaltend eine Rahmenöffnung, eine Keramik und einen Metallgehalt;
wobei die Elektrode (107) die Rahmenöffnung durchstößt,
wobei der Metallgehalt des Rahmens radial nach außen zunimmt.

13. Das Verfahren nach Anspruch 10, wobei das Gehäuse eine Keramik beinhaltet.

14. Das Verfahren nach einem der Ansprüche 10 bis 13, wobei der Verschluss elektrisch leitfähig ist.

15. Vorrichtung erhalten durch das Verfahren nach einem der Ansprüche 10 bis 14.

16. Verwendung einer Elektrode (107) zu einem Herstellen eines kabellosen Herzschrittmachers, wobei die Elektrode (107) ein Cermet beinhaltet

wobei die Elektrode (107) einstückig ausgebildet ist,
wobei die Elektrode (107) eine Kontaktoberfläche (108) beinhaltet,
wobei die Kontaktoberfläche (108) ausgebildet ist zu einem Kontaktieren eines eukaryotischen Gewebes,
**dadurch gekennzeichnet dass** die Kontaktoberfläche (108) oder eine Teiloberfläche (501, 502) der Kontaktoberfläche (108) oder beide eine offene Porendichte in einem Bereich von 1000 bis 80000 ppi hat.

**Claims**

1. A device (100) including a hollow body (101), an inner volume (102), and an ambient volume (103);

a) the inner volume (102) including an electronic component (104);
b) the hollow body (101)

i) enclosing the inner volume (102),
ii) including a first component (105), a second component (106), and an electrode (107);

c) the first component (105) being electrically conductive;
d) the second component (106) electrically insulating the electrode (107) from the first component (105);
e) the electrode (107)

i) including a cermet,
ii) electrically conductively connecting the inner volume (102) to the ambient volume (103),
iii) including a contact surface (108);

f) the contact surface (108)

i) being designed to contact a eukaryotic tissue,
ii) having a maximum distance from the electronic component (104) of less than 80 mm;

the electrode (107) being designed as a single piece;
the device (100) including the electronic component (104), the first component (105), the second component (106), and the electrode (107);
**characterized in that** the contact surface (108) or a partial surface (501, 502) of the contact surface (108) or both has/have an open pore density in a range of 1000 to 80,000 ppi.

2. The device (100) according to claim 1, wherein the electronic component (104) is an electric pulse generator.

3. The device (100) according to either of the preceding claims, wherein the electrode (107) is designed as a rigid body.

4. The device (100) according to any of the preceding claims, wherein the cermet includes a metal to at least 25 vol.% based on the volume of the electrode (107).

5. The device (100) according to any of the preceding claims, wherein the second component (106) includes a ceramic.

6. The device (100) according to any of the preceding claims, wherein the contact surface (108) includes an electrically conductive partial surface,
wherein the electrically conductive partial surface has an outer surface area of less than 25 mm$^2$.

7. The device (100) according to any of the preceding claims, wherein the contact surface (108) includes an electrically conductive partial surface (501),
wherein an inner surface area of the electrically conductive partial surface (501) is at least twice as large as the outer surface area of the electrically conductive partial surface (501).

8. The device (100) according to any of the preceding claims, wherein the contact surface (108) or a partial surface (501, 502) of the contact surface (108) or both has/have an average roughness in a range of 0.2 to 8 $\mu$m.

9. The device (100) according to any of the preceding claims, wherein the hollow body (101) is a therapy device.

10. A method for producing a device (100), including the following as method steps:

   a) providing a housing, including a housing opening;
   b) introducing an electronic component (104) into the housing through the housing opening;
   c) closing the housing opening with a closure;
   d) connecting the housing to an electrode (107);
   the electrode (107) including a cermet,
   the electrode (107) electrically conductively connecting the electronic component to an environment of the housing,
   the electrode (107) including a contact surface (108),
   the contact surface (108) being designed to contact a eukaryotic tissue,
   the contact surface (108) having a maximum distance from the electronic component (104) of less than 80 mm,
   **characterized in that** the method includes a further method step in which an open pore density of the contact surface (108) or a partial surface of the contact surface (108) or both is increased by etching the corresponding surface.

11. The method according to claim 10, wherein the housing is electrically conductive;

   wherein connecting the housing to the electrode (107) includes the following as substeps:

   a) connecting the housing to a connection flange (110), including a flange opening;
   b) introducing a frame, including a ceramic and a frame opening, into the flange opening;

   wherein the electrode (107) penetrates the frame opening.

12. The method according to claim 10, wherein the housing is electrically conductive;

   wherein connecting the housing to the electrode (107) includes, as substeps, connecting the housing to a frame including a frame opening, a ceramic and a metal content;
   wherein the electrode (107) penetrates the frame opening,
   wherein the metal content of the frame increases radially outward.

13. The method according to claim 10, wherein the housing includes a ceramic.

14. The method according to any of claims 10 to 13, wherein the closure is electrically conductive.

15. A device obtained by the method according to any of claims 10 to 14.

16. A use of an electrode (107) for establishing a wireless cardiac pacemaker, the electrode (107) including a cermet,

the electrode (107) being designed as a single piece,
the electrode (107) including a contact surface (108),
the contact surface (108) being designed to contact a eukaryotic tissue,
**characterized in that** the contact surface (108) or a partial surface (501, 502) of the contact surface (108) or both has/have an open pore density in a range of 1000 to 80,000 ppi.

## Revendications

1. Dispositif (100) contenant un corps creux (101), un volume interne (102) et un volume environnant (103) ; dans lequel

   a) le volume interne (102) contient un composant électronique (104) ;
   b) dans lequel le corps creux (101)

      i) entoure le volume interne (102),
      ii) contient un premier composant (105), un second composant (106) et une électrode (107) ;

   c) le premier composant (105) est électriquement conducteur ;
   d) le second composant (106) isole électriquement l'électrode (107) du premier composant (105) ;
   e) l'électrode (107)

      i) contient un cermet,
      ii) relie de manière électriquement conductrice le volume interne (102) au volume environnant (103),
      iii) contient une surface de contact (108) ;

   f) la surface de contact (108)

      i) est conçue pour une mise en contact avec un tissu eucaryote,
      ii) présente une distance maximale par rapport au composant électronique (104) inférieure à 80 mm ;

   dans lequel l'électrode (107) est conçue d'une seule pièce ;
   dans lequel le dispositif (100) contient le composant électronique (104), le premier composant (105), le second composant (106) et l'électrode (107) ;
   **caractérisé en ce que** la surface de contact (108) ou une surface partielle (501, 502) de la surface de contact (108) ou les deux surfaces présentent une densité de pores ouverts dans une plage de 1 000 à 80 000 ppi.

2. Dispositif (100) selon la revendication 1, dans lequel le composant électronique (104) est un générateur d'impulsions électrique.

3. Dispositif (100) selon l'une des revendications précédentes, dans lequel l'électrode (107) est conçue comme un corps rigide.

4. Dispositif (100) selon l'une des revendications précédentes, dans lequel le cermet contient un métal à au moins 25 % en volume sur la base du volume de l'électrode (107).

5. Dispositif (100) selon l'une des revendications précédentes, dans lequel le second composant (106) contient une céramique.

6. Dispositif (100) selon l'une des revendications précédentes, dans lequel la surface de contact (108) contient une surface partielle électriquement conductrice,
   dans lequel la surface partielle électriquement conductrice présente une surface externe inférieure à 25 mm$^2$.

7. Dispositif (100) selon l'une des revendications précédentes, dans lequel la surface de contact (108) contient une surface partielle (501) électriquement conductrice,
   dans lequel une surface interne de la surface partielle (501) électriquement conductrice est au moins deux fois plus grande que la surface externe de la surface partielle (501) électriquement conductrice.

**8.** Dispositif (100) selon l'une des revendications précédentes, dans lequel la surface de contact (108) ou une surface partielle (501, 502) de la surface de contact (108) ou les deux surfaces présentent une rugosité moyenne dans une plage de 0,2 à 8 μm.

**9.** Dispositif (100) selon l'une des revendications précédentes, dans lequel le corps creux (101) est un appareil thérapeutique.

**10.** Procédé pour la fabrication d'un dispositif (100), contenant en tant qu'étapes de procédé

> a) la fourniture d'un boîtier, contenant une ouverture de boîtier ;
> b) l'introduction d'un composant électronique (104) dans le boîtier à travers l'ouverture de boîtier ;
> c) la fermeture de l'ouverture de boîtier avec une fermeture ;
> d) la liaison du boîtier à une électrode (107) ;
> dans lequel l'électrode (107) contient un cermet,
> dans lequel l'électrode (107) relie de manière électriquement conductrice le composant électronique à un environnement du boîtier,
> dans lequel l'électrode (107) contient une surface de contact (108),
> dans lequel la surface de contact (108) est conçue pour une mise en contact avec un tissu eucaryote,
> dans lequel la surface de contact (108) présente une distance maximale par rapport au composant électronique (104) inférieure à 80 mm,
> **caractérisé en ce que** le procédé contient une étape de procédé supplémentaire, dans laquelle une densité de pores ouverts de la surface de contact (108) ou d'une surface partielle de la surface de contact (108) ou des deux surfaces est augmentée par une gravure de la surface correspondante.

**11.** Procédé selon la revendication 10, dans lequel le boîtier est électriquement conducteur ;

> dans lequel la liaison du boîtier à l'électrode (107) contient, en tant que sous-étapes
>
> > a) la liaison du boîtier à une bride de raccordement (110), contenant une ouverture de bride ;
> > b) l'introduction d'un cadre, contenant une céramique et une ouverture de cadre, dans l'ouverture de bride ;
>
> dans lequel l'électrode (107) transperce l'ouverture de cadre.

**12.** Procédé selon la revendication 10, dans lequel le boîtier est électriquement conducteur ;

> dans lequel la liaison du boîtier à l'électrode (107) contient, en tant que sous-étapes une liaison du boîtier à un cadre, contenant une ouverture de cadre, une céramique et une teneur en métal ;
> dans lequel l'électrode (107) transperce l'ouverture de cadre,
> dans lequel la teneur en métal du cadre augmente radialement vers l'extérieur.

**13.** Procédé selon la revendication 10, dans lequel le boîtier contient une céramique.

**14.** Procédé selon l'une des revendications 10 à 13, dans lequel la fermeture est électriquement conductrice.

**15.** Dispositif obtenu par le biais du procédé selon l'une des revendications 10 à 14.

**16.** Utilisation d'une électrode (107) pour la fabrication d'un stimulateur cardiaque sans fil,

> dans lequel l'électrode (107) contient un cermet
> dans lequel l'électrode (107) est conçue d'une seule pièce,
> dans lequel l'électrode (107) contient une surface de contact (108),
> dans lequel la surface de contact (108) est conçue pour une mise en contact avec un tissu eucaryote,
> **caractérisée en ce que** la surface de contact (108) ou une surface partielle (501, 502) de la surface de contact (108) ou les deux surfaces présentent une densité de pores ouverts dans une plage de 1 000 à 80 000 ppi.

Fig. 1

100

## Fig. 2a

<u>100</u>

103   101

106
107
108
204
205
206

109   203   202   104   105
      102

201

## Fig. 2b

107
204
205
206

106

Fig. 3

100

Fig. 4

<u>100</u>

Fig. 5

<u>100</u>

Fig. 6a

Fig. 6b

Fig. 6c

Fig. 7a

Fig. 7b

Fig. 8

800

Fig. 9

<u>100</u>

**IN DER BESCHREIBUNG AUFGEFÜHRTE DOKUMENTE**

**In der Beschreibung aufgeführte Patentdokumente**

- EP 1714670 A1

- US 20130338750 A1